(19) **Europäisches Patentamt / European Patent Office / Office européen des brevets**

(11) **EP 4 779 386 A1**

(12) **EUROPEAN PATENT APPLICATION**
published in accordance with Art. 153(4) EPC

(43) Date of publication:
**22.07.2026 Bulletin 2026/30**

(21) Application number: **24865366.9**

(22) Date of filing: **06.09.2024**

(51) International Patent Classification (IPC):
*G02B 21/34* (2006.01)    *G01J 3/52* (2006.01)
*G01N 21/01* (2006.01)    *G01N 21/27* (2006.01)
*G02B 21/00* (2006.01)

(52) Cooperative Patent Classification (CPC):
G01J 3/52; G01N 21/01; G01N 21/27; G01N 33/48;
G01N 33/483; G02B 21/00; G02B 21/34

(86) International application number:
**PCT/JP2024/031975**

(87) International publication number:
**WO 2025/057866 (20.03.2025 Gazette 2025/12)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(30) Priority:  **11.09.2023  JP 2023146990
07.05.2024  JP 2024075377**

(71) Applicant: **Dai Nippon Printing Co., Ltd.
Tokyo 162-8001 (JP)**

(72) Inventors:
• **NISHIO Naoko
Tokyo 162-8001 (JP)**
• **MURAYAMA Yusuke
Tokyo 162-8001 (JP)**
• **MORO Miharu
Tokyo 162-8001 (JP)**
• **TANAKA Suguru
Tokyo 162-8001 (JP)**
• **YASUI Ryoma
Tokyo 162-8001 (JP)**
• **KOMATSU Haruna
Tokyo 162-8001 (JP)**
• **OGINO Yoshihiko
Tokyo 162-8001 (JP)**
• **SUGIYAMA Tohru
Tokyo 162-8001 (JP)**

(74) Representative: **Müller-Boré & Partner
Patentanwälte PartG mbB
Friedenheimer Brücke 21
80639 München (DE)**

(54) **COLOR CORRECTION CHART**

(57)    A color correction chart (10) includes a substrate (11) and a color patch (12a) formed on the substrate (11), wherein the color patch (12a) includes a first color, and the first color has a coordinate point within a region (L1) bounded by three points (0.200, 0.070), (0.450, 0.280), and (0.310, 0.340) on an xy chromaticity diagram.

**EP 4 779 386 A1**

FIG. 2A

**Description**

Technical Field

The present disclosure relates to a color correction chart.

Background Art

In the field of pathology, tissues and cells have been examined. In this process, staining reagents are used to stain tissues and cells. Lesions are then distinguished on the basis of the color changes in the stained tissues and cells to diagnose diseases.

Stained tissues and cells may be observed directly under a microscope. Alternatively, stained tissues or cells may be imaged using a CCD camera attached to a microscope and then output to a display for observation. Furthermore, stained tissues or cells may be stored as digital images captured using a microscope for pathology slides or a glass-slide scanner. In this case, the digital images may be utilized for telemedicine or AI-based diagnosis.

When stained tissues or cells are imaged to obtain digital images, the colors may vary depending on the imaging device used. Therefore, a color correction tool is used to correct color differences between imaging devices.

Citation List

Patent Literature

PTL 1: U.S. Patent No. 10241310

Summary of Invention

Technical Problem

PTL 1 discloses a method for performing correction using color patches formed by using biomimetic substances and stains used for pathological staining. However, even with the same stain, differences in the stained matrix commonly result in variations in the color of light transmitted through or reflected from a stained preparation, or the spectrum of the light transmitted through or reflected from the stained preparation. This is because there are differences in the chemical bonding state between the stain and the matrix. Such differences arise due to factors, such as interactions between the matrix and the stain or other additives, and differences in the degree to which the stain is dispersed in the matrix, which arise in the process where the stain is fixed to the matrix.

The present disclosure provides a color correction chart that can enhance the color-correction effect for pathological specimen images.

Disclosure of Invention

An embodiment of the present disclosure relates to the following [1] to [15].

[1] A color correction chart comprising: a substrate; and a color patch formed on the substrate, wherein the color patch includes a first color, and the first color has a coordinate point within a region bounded by three points (0.200, 0.070), (0.450, 0.280), and (0.310, 0.340) on an xy chromaticity diagram.

[2] The color correction chart according to [1], wherein the first color has a coordinate point within a region bounded by five points (0.310, 0.340), (0.350, 0.300), (0.350, 0.200), (0.260, 0.150), and (0.280, 0.250) on the xy chromaticity diagram.

[3] The color correction chart according to [1] or [2], wherein the first color has a coordinate point within a region bounded by four points (0.311, 0.334), (0.347, 0.210), (0.270, 0.162), and (0.292, 0.216) on the xy chromaticity diagram.

[4] The color correction chart according to [1], wherein the first color has a coordinate point within a region bounded by three points (0.350, 0.300), (0.350, 0.200), and (0.380, 0.280) on the xy chromaticity diagram.

[5] The color correction chart according to any one of [1] to [3], further comprising a plurality of color patches formed on the substrate, wherein the color patches each have a color different from the first color, the color patches each include a different color, and the color patches have color coordinate points within the region bounded by three points (0.200, 0.070), (0.450, 0.280), and (0.310, 0.340) on the xy chromaticity diagram.

[6] The color correction chart according to [5], wherein the color patches have color coordinate points within a region bounded by five points (0.310, 0.340), (0.350, 0.300), (0.350, 0.200), (0.260, 0.150), and (0.280, 0.250) on the xy chromaticity diagram.

[7] The color correction chart according to [5] or [6], wherein the color patches have color coordinate points within a region bounded by four points (0.311, 0.334), (0.347, 0.210), (0.270, 0.162), and (0.292, 0.216) on the xy chromaticity diagram.

[8] The color correction chart according to any one of [2] to [4], further comprising an additional color patch formed on the substrate, wherein the additional color patch has a color coordinate point within a region bounded by three points (0.350, 0.300), (0.350, 0.200), and (0.380, 0.280) on the xy chromaticity diagram.

[9] The color correction chart according to any one of [1] to [8], wherein the first color has a coordinate point outside a white region defined by a blackbody locus of 5000 K or higher and 9300 K or lower and a color deviation of $d_{UV} = \pm 0.005$ from the blackbody locus.

[10] The color correction chart according to any one of [1] to [9], wherein a spectrum of the first color has a peak wavelength of 520 nm or more and 560 nm or less, a point at which the spectrum rises toward a long-wavelength side from the peak wavelength is in a range of 520 nm or more and 560 nm or less, and an increase in spectral transmittance in a range of 560 nm or more and 620 nm or less is 40 %T or less.

[11] The color correction chart according to [10], wherein the spectral transmittance at the peak wavelength is 0.5 %T or more and 45 %T or less.

[12] The color correction chart according to any one of [1] to [11], wherein an amount of color change $\Delta E^*ab$ of the first color of the color patch is 2.5 or less after irradiation with light from a white LED light source having a color rendering of $Ra \geq 90$ and $R9 \geq 80$ and having a spectral distribution simulating that of standard illuminant D65 is performed at an irradiance of 50 W/m$^2$ to 65 W/m$^2$ on an irradiated object and a cumulative light dose of 400 W/m$^2$·h.

[13] The color correction chart according to any one of [1] to [12], wherein at least one of an amount of change in transmittance (%T) at 500 nm and an amount of change in transmittance (%T) at 520 nm in a spectrum of the first color of the color patch is 2.0 %T or less after irradiation with light from a white LED light source having a color rendering of $Ra \geq 90$ and $R9 \geq 80$ and having a spectral distribution simulating that of standard illuminant D65 is performed at an irradiance of 50 W/m$^2$ to 65 W/m$^2$ on an irradiated object and a cumulative light dose of 435 W/m$^2$·h.

[14] The color correction chart according to any one of [1] to [13], wherein at least one of a rate of change in transmittance (%T) at 500 nm and a rate of change in transmittance (%T) at 520 nm in a spectrum of the first color of the color patch is 10% or less after irradiation with light from a white LED light source having a color rendering of $Ra \geq 90$ and $R9 \geq 80$ and having a spectral distribution simulating that of standard illuminant D65 is performed at an irradiance of 50 W/m$^2$ to 65 W/m$^2$ on an irradiated object and a cumulative light dose of 435 W/m$^2$·h.

[15] The color correction chart according to any one of [1] to [14], wherein the substrate includes a dye-fixing layer.

According to this embodiment, the color-correction effect for pathological specimen images can be enhanced.

Brief Description of Drawings

[Fig. 1] Fig. 1 is a plan view illustrating a color correction chart according to one embodiment.

[Fig. 2A] Fig. 2A is an xy chromaticity diagram illustrating the color gamut of a color correction chart according to one embodiment.

[Fig. 2B] Fig. 2B is an xy chromaticity diagram illustrating the color gamut of the color correction chart according to one embodiment, wherein a predetermined white region is superimposed on this diagram.

[Fig. 2C] Fig. 2C is an xy chromaticity diagram illustrating the color gamut of the color correction chart according to one embodiment, wherein plotted points of pathological specimens are superimposed on this diagram.

[Fig. 3A] Fig. 3A is an xy chromaticity diagram illustrating the color gamut of a color correction chart according to another example.

[Fig. 3B] Fig. 3B is an xy chromaticity diagram illustrating the color gamut of the color correction chart according to another example, wherein a predetermined white region is superimposed on this diagram.

[Fig. 3C] Fig. 3C is an xy chromaticity diagram illustrating the color gamut of the color correction chart according to another example, wherein plotted points of pathological specimens are superimposed on this diagram.

[Fig. 4] Fig. 4 is a graph illustrating an example of the spectrum indicated by the color correction chart according to one embodiment.

[Fig. 5] Fig. 5 is an xy chromaticity diagram illustrating the color gamut of the color correction chart according to another example.

[Fig. 6A] Fig. 6A is a graph illustrating the spectra of color patches of first to seventh colors of a color correction chart according to Example 1.

[Fig. 6B] Fig. 6B is a graph illustrating the spectra of color patches of first to eleventh colors of a color correction chart according to Example 2.

[Fig. 6C] Fig. 6C is a graph illustrating the spectra of color patches of first to fourteenth colors of a color correction chart according to Example 4.

[Fig. 7] Fig. 7 includes photographs illustrating a specimen of an actual stained pathological cell preparation.

[Fig. 8] Figs. 8A to 8D are graphs respectively illustrating changes in the color difference ΔE*ab before and after color correction of the images of cytoplasm, extracellular matrix (ECM), red blood cells (RBCs), and cell nuclei.

[Fig. 9] Figs. 9A to 9C are graphs illustrating changes in the color difference ΔE*ab before and after color correction of the images of cytoplasm, extracellular matrix, and cell nuclei obtained using different WSI scanners.

[Fig. 10] Fig. 10 includes graphs illustrating the spectra before and after lightfastness evaluation for each of color patches a to d and specimens i and j.

Description of Embodiments

Each embodiment will be described below in detail with reference to the drawings. The figures shown below are schematically illustrated. Accordingly, the size and shape of each part are appropriately exaggerated for ease of understanding. The present disclosure can be implemented with appropriate modifications, as long as such modifications do not depart from the technical concept. In the figures shown below, identical parts are denoted by the same reference signs, and detailed descriptions of those parts may be partially omitted. The numerical values, such as the dimensions of members, and the names of materials described in this description are merely examples in the embodiments and are not limited thereto; the numerical values and the names of materials can be appropriately selected and used.

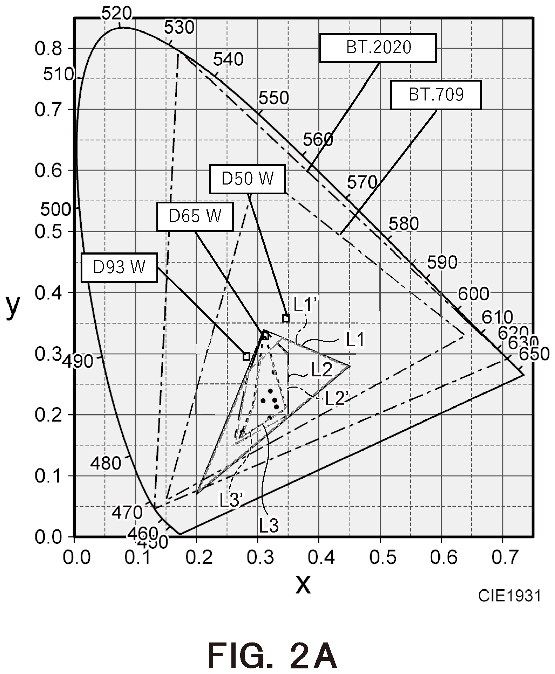

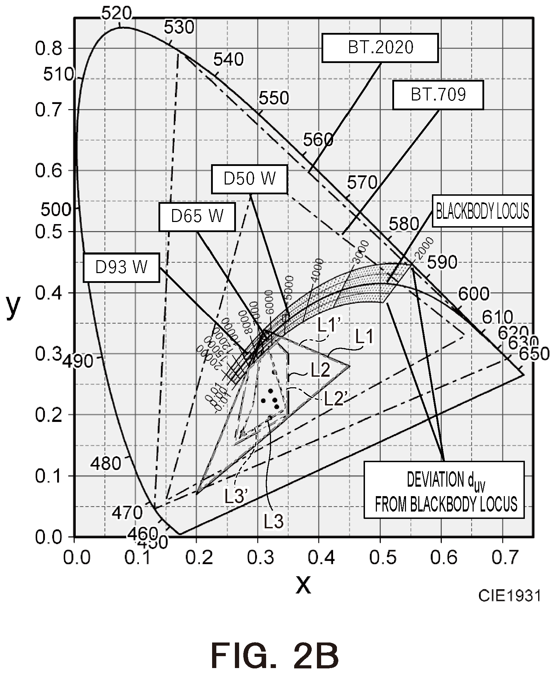

In this description, the term "color gamut" refers to a specific range within the visible region and can be represented using an xy chromaticity diagram of the CIE (International Commission on Illumination) XYZ color space (CIE 1931 XYZ color space), for example, as illustrated in Figs. 2A to 2C. In the xy chromaticity diagram, the color gamut can be represented as a polygon formed by connecting, with straight lines, chromaticity coordinates serving as vertices corresponding to the respective colors. The color gamut is conventionally defined by various color gamut standards. In the video industry, which includes imaging devices, standards covering wide color gamuts, such as BT.709 and BT.2020, as illustrated in Figs. 2A to 2C, are used. In the xy chromaticity diagrams illustrated in Figs. 2A and 2B, the CIE standard illuminant D65 serving as a white point is plotted as a square (□). In the xy chromaticity diagrams illustrated in Figs. 2A and 2B, the white reference illuminant D50 in the printing industry and the white reference illuminant D93 in the broadcasting industry according to the NTSC-J standard are each plotted as a square (□).

(Configuration of Color Correction Chart)

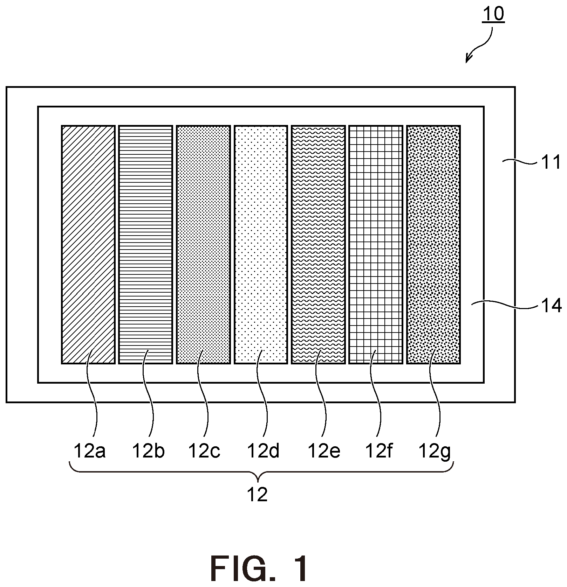

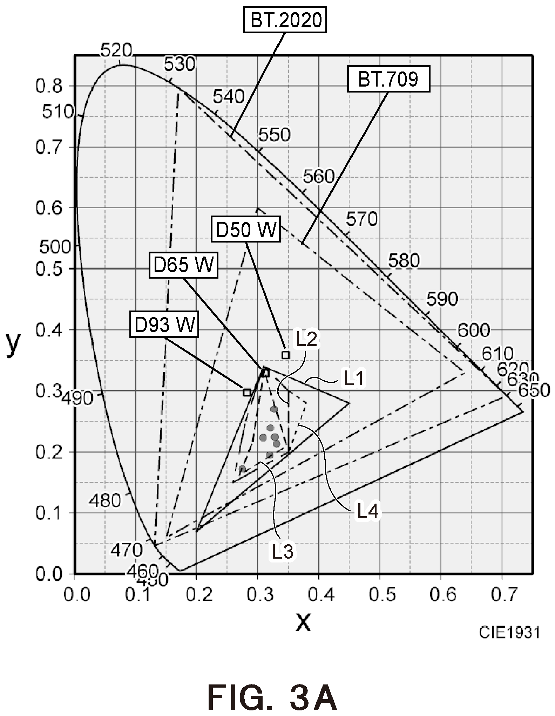

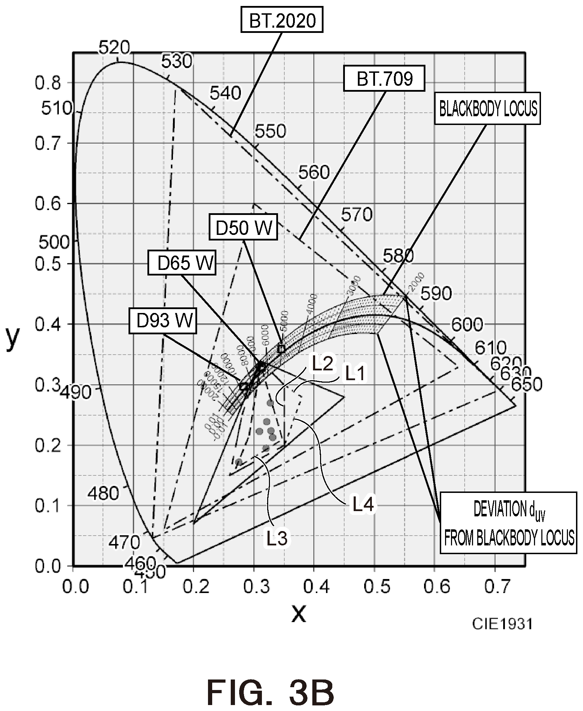

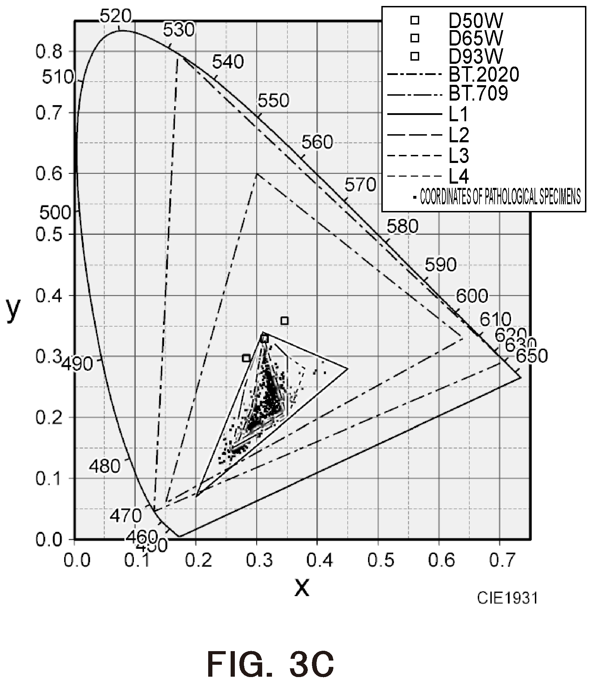

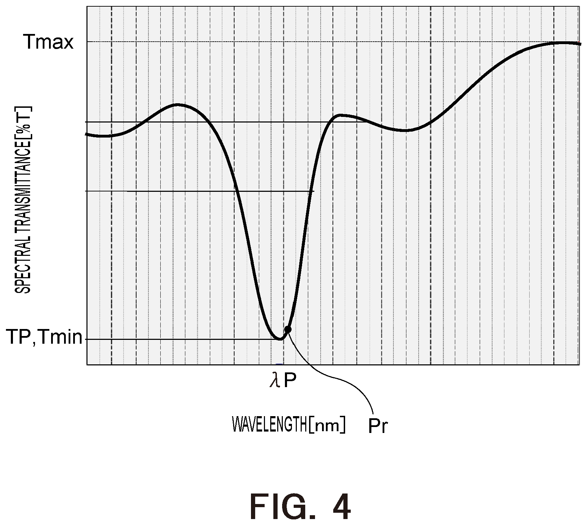

A color correction chart according to this embodiment will be described with reference to Figs. 1 to 4. Fig. 1 is a schematic plan view illustrating an example of the color correction chart according to this embodiment. Figs. 2A to 2C are the xy chromaticity diagrams each illustrating the color gamut of the color correction chart according to this embodiment. Figs. 3A to 3C are the xy chromaticity diagrams each illustrating the color gamut of a color correction chart according to another example. Fig. 4 is a graph illustrating an example of the spectrum indicated by the color correction chart according to this embodiment.

A color correction chart 10 according to this embodiment is used for performing color correction on stained pathological specimen images. The color correction chart 10 includes a substrate 11 and a color patch group 12 formed on the substrate 11. The color correction chart may also be referred to as a color chart.

The color patch group 12 includes seven color patches: a first color patch 12a, a second color patch 12b, a third color patch 12c, a fourth color patch 12d, a fifth color patch 12e, a sixth color patch 12f, and a seventh color patch 12g. Hereinafter, the first color patch 12a, the second color patch 12b, the third color patch 12c, the fourth color patch 12d, the fifth color patch 12e, the sixth color patch 12f, and the seventh color patch 12g are also referred to simply as color patches 12a to 12g. The color patches 12a to 12g are arranged in a pattern. The color patch group 12 includes at least one color patch, and preferably includes three or more color patches. The color patches 12a to 12g on the substrate 11 are surrounded by a light-shielding portion 14. The color patches may also be referred to as color regions.

Referring to Fig. 2A, the color coordinate points of the color patches 12a to 12g are located within a region bounded by three points (0.200, 0.070), (0.450, 0.280), and (0.310, 0.340) on the xy chromaticity diagram. In other words, the color coordinate points of the color patches 12a to 12g are located in a first color gamut L1 bounded by a triangle formed by straight lines connecting the coordinates (0.200, 0.070), (0.450, 0.280), and (0.310, 0.340) on the xy chromaticity diagram. In this description, the phrase "located within a region bounded by (three or more) points" includes not only coordinate points located inside the sides of the polygon formed by the plurality of points, but also coordinate points located on the sides of the polygon or the vertices of the polygon.

Referring to Fig. 2B, the first color gamut L1 may be a first modified color gamut L1' that is located within a coordinate range on the xy chromaticity diagram and excludes a predetermined white region. The predetermined white region may be, for example, the region defined by a blackbody locus of 5000 K or higher and 9300 K or lower and a color deviation of $d_{UV} = \pm 0.005$ from the blackbody locus.

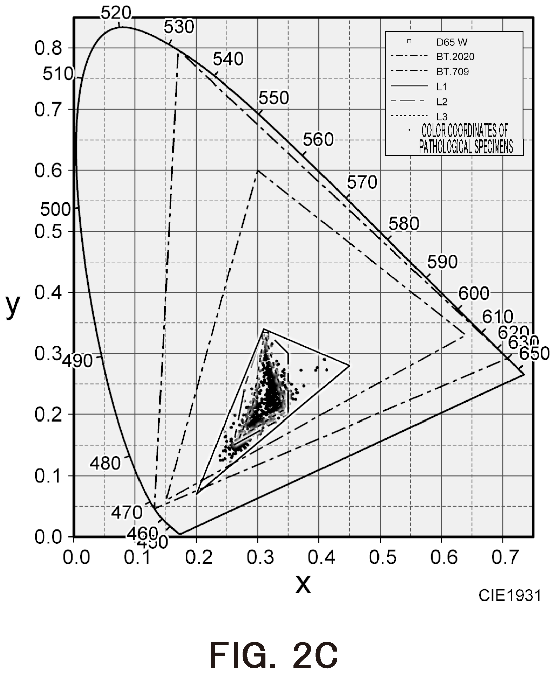

Referring to Fig. 2C, the first color gamut L1 is a region that includes colors at 759 points (hereinafter referred to as "colors at extraction points") extracted from hematoxylin and eosin (HE)-stained specimens from different types of organs, tissues, or cells. Of the 759 extraction points, 710 points are included in the first modified color gamut L1', which excludes the white region. When an object to be corrected contains many colors belonging to the first modified color gamut L1', color correction can be performed more effectively by using patches having colors included in the first modified color gamut L1'. Specifically, the extraction points are extracted as follows. Eighty three specimens were prepared from 14 types of organs, and a total of 759 extraction points were determined by randomly selecting multiple locations within a region containing cells of interest in each specimen. The colors at the extraction points are specified as follows. Each stained specimen was sandwiched between a glass slide and a cover glass to prepare a specimen slide. Next, the specimen was observed using a microscope (MX61L, light source LG-PS2) available from Olympus Corporation. During observation, the spectrum of the specimen was measured using a spectroradiometer (SR-5000HM, available from Topcon Technohouse Corporation) connected to the microscope. The spectrum of the light from the light source LG-PS2 used was also measured. Next, the spectral transmittance of the specimen was obtained on the basis of the spectrum of the light from the light source LG-PS2 that has passed through the specimen, and the spectrum of the light source LG-PS2 itself. Furthermore, based on the obtained spectral transmittance of the specimen, the coordinate points on the xy chromaticity diagram are calculated using the spectral distribution data of the CIE standard illuminant D65. The light source used is not limited to the above light source, and any light source can be used as long as it has sufficient intensity in the entire visible light range (380 nm to 780 nm).

When the color coordinate points of the color patches 12a to 12g are located within the first color gamut L1 on the xy chromaticity diagram, the colors of the color patches 12a to 12g of the color correction chart 10 can be close to colors characteristic of stained pathological cell preparations. The stained pathological cell preparations may be hematoxylin and eosin (HE)-stained preparations. When the colors of the color patches 12a to 12g are close to colors characteristic of stained pathological cell preparations, color correction of an imaging device can be accurately performed using the color correction chart 10.

Referring to Fig. 2A, the color coordinate points of the color patches 12a to 12g are preferably located within a region bounded by five points (0.310, 0.340), (0.350, 0.300), (0.350, 0.200), (0.260, 0.150), and (0.280, 0.250) on the xy chromaticity diagram. In other words, the color coordinate points of the color patches 12a to 12g are preferably located in a second color gamut L2 bounded by a pentagon formed by straight lines connecting the coordinates (0.310, 0.340), (0.350, 0.300), (0.350, 0.200), (0.260, 0.150), and (0.280, 0.250) on the xy chromaticity diagram in this order. The second color gamut L2 is completely included in the first color gamut L1. When the color coordinate points of the color patches 12a to 12g are located in the second color gamut L2 on the xy chromaticity diagram, color correction of an imaging device can be performed more accurately using the color correction chart 10.

Referring to Fig. 2B, the second color gamut L2 may be a second modified color gamut L2' that is located within a coordinate range on the xy chromaticity diagram and excludes a predetermined white region. The predetermined white region may be, for example, the region defined by a blackbody locus of 5000 K or higher and 9300 K or lower and a color deviation of $d_{UV} = \pm0.005$ from the blackbody locus.

Referring to Fig. 2C, the second color gamut L2 is a region that includes colors at 633 points among the colors at the 759 extraction points (excluding points on the boundary lines). The density of the colors at the extraction points included in the second color gamut L2 is 1.87 times that at the extraction points included in the first color gamut L1. The density is calculated by dividing the number of colors at the extraction points included in each of the first color gamut L1 and the second color gamut L2 by the area of the corresponding color gamut on the xy chromaticity diagram. When the color coordinate points of the color patches 12a to 12g are included in the second color gamut L2, the images of stained pathological cell preparations can be corrected more efficiently. Of the 633 extraction points, 584 points are included in the second modified color gamut L2', which excludes the white region.

Referring to Fig. 2A, the color coordinate points of the color patches 12a to 12g are preferably located within a region bounded by four points (0.311, 0.334), (0.347, 0.210), (0.270, 0.162), and (0.292, 0.216) on the xy chromaticity diagram. In other words, the color coordinate points of the color patches 12a to 12g are preferably located in a third color gamut L3 bounded by a rectangle formed by straight lines connecting the coordinates (0.311, 0.334), (0.347, 0.210), (0.270, 0.162), and (0.292, 0.216) on the xy chromaticity diagram in this order. The third color gamut L3 is completely included in the second color gamut L2. When the color coordinate points of the color patches 12a to 12g are located in the third color gamut L3 on the xy chromaticity diagram, color correction of an imaging device can be performed more accurately using the color correction chart 10.

Referring to Fig. 2B, the third color gamut L3 may be a third modified color gamut L3' that is located within a coordinate range on the xy chromaticity diagram and excludes a predetermined white region. The predetermined white region may be, for example, the region defined by a blackbody locus of 5000 K or higher and 9300 K or lower and a color deviation of $d_{UV} = \pm0.005$ from the blackbody locus.

Referring to Fig. 2C, the third color gamut L3 is a region that includes characteristic colors selected from the colors at the above 759 extraction points by using a statistical method. When the color coordinate points of the color patches 12a to 12g are included in the third color gamut L3, the images of stained pathological cell preparations can be corrected more efficiently.

The chromaticity coordinates of the color patches on the xy chromaticity diagram are calculated from the following equations (collectively referred to as Equation A).

[Math. 1]

$$X = k \int_{\lambda} \tau(\lambda)S(\lambda)\bar{x}(\lambda)\,d\lambda$$

$$Y = k \int_{\lambda} \tau(\lambda)S(\lambda)\bar{y}(\lambda)\,d\lambda$$

$$Z = k \int_{\lambda} \tau(\lambda)S(\lambda)\bar{z}(\lambda)\,d\lambda$$

$$k = 100 \Big/ \sum_{\lambda} S(\lambda)\bar{y}(\lambda)\Delta\lambda$$

[Math. 2]

$$x = \frac{X}{X+Y+Z} \quad y = \frac{Y}{X+Y+Z}$$

$$z = \frac{Z}{X+Y+Z}$$

$\tau(\lambda)$: spectral transmittance
$S(\lambda)$: relative spectral distribution of illumination light
$\overline{x}(\lambda)$, $\overline{y}(\lambda)$, $\overline{z}(\lambda)$: color-matching functions for the CIE 1931 standard colorimetric observer
k: normalization factor
* wavelength range used $\lambda$ = 400 to 700 nm
* $\Delta\lambda$ = 1 nm and 5 nm
* A D65 light source is used for illumination

The above equations are specified in JIS Z8781.

Alternatively, referring to Figs. 3A to 3C, the color coordinate points of the color patches 12a to 12g may be located within a region bounded by three points (0.350, 0.300), (0.350, 0.200), and (0.380, 0.280) on the xy chromaticity diagram. In other words, the color coordinate points of the color patches 12a to 12g may be located in a fourth color gamut L4 bounded by a triangle formed by straight lines connecting the coordinates (0.350, 0.300), (0.350, 0.200), and (0.380, 0.280) on the xy chromaticity diagram. The fourth color gamut L4 is completely included in the first color gamut L1, but is not included in the second color gamut L2 or the third color gamut L3.

The fourth color gamut L4 is a color gamut that includes a color similar to that of red blood cells. Accordingly, when the color coordinate points of the color patches 12a to 12g are located in the fourth color gamut L4 on the xy chromaticity diagram, the accuracy of color correction particularly for regions other than cell nuclei can be improved.

The color correction chart 10 according to this embodiment has spectral characteristics as illustrated in Fig. 4. The transmission spectrum of each of the color patches 12a to 12g has a peak wavelength at a predetermined position. In the spectrum of the color correction chart 10 according to this embodiment, the spectral shapes of the color patches 12a to 12g approximate those of actual stained pathological cell preparations. This feature suppresses deviations between the colors of the color patches 12a to 12g and the colors of the stained pathological cell preparation that may occur due to differences in the environment in which the color correction chart 10 is used, such as variations in the light source. As a result, metamerism (conditional color matching) is unlikely to occur when the color correction chart 10 is used.

The spectrum of each of the color patches 12a to 12g has a peak wavelength. In other words, the color patches 12a to 12g each exhibit a waveform as illustrated in Fig. 4 and each has a peak wavelength $\lambda P$ that indicates low transmittance. The term "peak wavelength $\lambda P$" refers to the wavelength at which the relative transmittance reaches its minimum when the transmittance corresponding to the maximum measured transmittance in the spectrum is taken as 100%. In other words, as illustrated in Fig. 4, the peak wavelength $\lambda P$ is defined as the wavelength at which the relative transmittance reaches the minimum transmittance Tmin when the maximum measured transmittance Tmax in the spectrum is specified and taken as 100%. The spectra of the color patches 12a to 12g are obtained by measuring the transmittance at 1 nm intervals in the visible light range of 380 nm to 780 nm using a spectroradiometer (SR-5000HM, available from Topcon Technohouse Corporation), with a colorless (transparent) white region used as the background. To calculate the coordinates of each color on the xy chromaticity diagram, the spectral distribution data of the known CIE standard illuminant D65 are used.

Specifically, the peak wavelength $\lambda P$ of the spectrum of each of the color patches 12a to 12g may be 520 nm or more and 560 nm or less. The spectral transmittance TP at the peak wavelength $\lambda P$ may be 0.5 %T or more (or the measurement limit or more), may be 1 %T or more, may be 2 %T or more, or may be 3 %T or more. The spectral transmittance TP at the peak wavelength $\lambda P$ may be 45 %T or less, may be 40 %T or less, may be 35 %T or less, or may be 30 %T or less. When the peak wavelength $\lambda P$ and the spectral transmittance TP at the peak wavelength $\lambda P$ of the spectrum of each of the color patches 12a to 12g are in the above ranges, the spectral shapes of the color patches 12a to 12g can approximate those of actual stained pathological cell preparations, and metamerism is thus unlikely to occur when the color correction chart 10 is used.

In the spectrum of each of the color patches 12a to 12g, the peak wavelength $\lambda P$ may be 520 nm or more and 560 nm or less, as described above. A point Pr (Fig. 4) at which the spectrum rises toward the longer wavelength side from the peak wavelength $\lambda P$ is preferably located in the range of 520 nm or more and 560 nm or less. The point Pr at which the spectrum rises refers to a point at which the spectral transmittance increases by 5 %T from the spectral transmittance TP at the peak wavelength λP, or a point of the minimum transmittance in a range of 520 nm or more and 560 nm or less. An increase in spectral transmittance in a wavelength range of 560 nm or more and 620 nm or less is preferably 40 %T or less. Specifically, when the spectral transmittance at a wavelength of 620 nm is denoted by T620 (%T) and the spectral transmittance at a wavelength of 560 nm is denoted by T560 (%T), the value of T620 - T560 is preferably 40 %T or less. When the value of T620 - T560 is 40 %T or less, the spectral shapes of the color patches 12a to 12g can approximate those of actual stained pathological cell preparations, and metamerism is thus unlikely to occur when the color correction chart 10 is used.

In the spectra of at least some of the color patches 12a to 12g, the value obtained by subtracting the spectral transmittance at a wavelength of 400 nm from that at a wavelength of 450 nm is preferably 4 %T or more. Specifically, when the spectral transmittance at a wavelength of 450 nm is denoted by T450 (%T) and the spectral transmittance at a wavelength of 400 nm is denoted by T400 (%T), the value of T450 - T400 is preferably 4 %T or more. In general, many LED light sources have a spectral peak around a wavelength of 450 nm. Accordingly, when an LED light source is used, the influence of blue light from the LED light source on the visual appearance can be reduced.

In the spectra of at least some of the color patches 12a to 12g, the spectral transmittance at a wavelength of 630 nm is preferably 35 %T or less. In this case, the color of cell nuclei with higher chroma can be reproduced.

The light-shielding portion 14 illustrated in Fig. 1 has a desired light-shielding property. Examples of the light-shielding portion 14 include a metal film, such as a chromium thin film, and a printed layer formed with black ink. The light-shielding portion 14 can be formed by a conventionally known method depending on the material used.

The substrate 11 is any substrate that can support the color patch group 12 and the light-shielding portion 14. The substrate 11 may be a transparent substrate having light transmittance. The substrate 11 may be of the same type as that used in conventionally known color charts. Specifically, the substrate 11 may be an inorganic substrate, such as a glass substrate, or a resin substrate. The resin substrate may be in the form of a plate, a film, or a sheet.

The substrate 11 may be a glass slide used for observation with a pathological imaging device, such as a microscope. The glass slide may be provided with a support portion for supporting a stained pathological cell preparation. The color patch group 12 and the support portion may be covered with a cover glass.

Alternatively, the color correction chart 10 and the glass slide may be provided separately. In this case, the color correction chart 10 may be slidable relative to the glass slide that supports a stained pathological cell preparation. This configuration allows the color correction chart to be imaged by a pathological imaging device under the same conditions as those during observation of the stained pathological cell preparation. Since the color correction chart 10 is not covered with a cover glass, contamination or scratches on the cover glass do not affect the color patch group 12. It is also possible to suppress color changes in the color patch group 12 that would otherwise occur when the color correction chart 10 is covered with a cover glass.

The substrate 11 may be made of a material in which dyes used for producing the color patches 12a to 12g can be dispersed. The substrate 11 may also be made of a material that can be chemically bonded to the dyes used for producing the color patches 12a to 12g. Examples of such chemical bonding include hydrogen bonding, ionic bonding, chelate bonding, covalent bonding, and bonding caused by dispersion forces or orientation forces.

The substrate 11 may include a dye-fixing layer. The dye-fixing layer is a layer that can be chemically bonded to the dyes used for producing the color patches 12a to 12g. Examples of the dye-fixing layer include those containing proteins, such as gelatin and collagen, and synthetic resins, such as nylon, polyester, and acrylic resins. Examples of the substrate 11 including the dye-fixing layer include a photomask drawing film, a prepress film, a phototooling film, a photographic paper. When the substrate 11 includes the dye-fixing layer, chemical bonding is formed between the substrate 11 and the dyes to improve fastness to moisture, friction, and light.

The dyes used for producing the color patches 12a to 12g may be water-soluble dyes. Examples of the water-soluble dyes include dyes used for textile applications, and food dyes. Examples of the types of dyes include acid dyes, direct dyes, cationic dyes, metal-complex acid dyes, acid mordant dyes, and reactive dyes. Examples of acid dyes and direct dyes include dyes known as Acid Yellow, Acid Violet, Acid Red, Acid Green, Acid Blue, Acid Black, Acid Orange, Acid Brown, Direct Red, Direct Black, Direct Orange, Direct Yellow, Direct Green, Direct Violet, Direct Brown, and Direct Blue. Examples of food dyes include dyes known as Food Red, Food Green, Food Blue, Food Yellow, Food Black, Food Brown, Food Violet, and Food Orange. Alternatively, examples of the dyes include mixtures of the above dyes, and dyes used under similar dyeing conditions and for similar dyeing applications. One type of dye may be used alone, or two or more types of dyes may be used as a mixture.

The use of the above materials for the substrate 11 and the dyes can suppress color changes (fading) in the color patches 12a to 12g caused by light from a light source even when the color correction chart 10 according to this embodiment is used for a long period of time or multiple times, as described below. As a result, even after long-term or multiple uses of the color correction chart 10, deterioration in color-correction accuracy can be suppressed, and a high color-correction effect can be maintained.

Specifically, in the color correction chart 10 according to this embodiment, the amount of color change ΔE*ab of the color patches 12a to 12g after long-term light irradiation is reduced. More specifically, the amount of color change ΔE*ab of the color patches 12a to 12g is preferably 2.5 or less after irradiation with light from a white LED light source having a color rendering of Ra ≥ 90 and R9 ≥ 80 and having a spectral distribution simulating that of standard illuminant D65 is performed at an irradiance of 50 W/m² to 65 W/m² on an irradiated object and a cumulative light dose of 400 W/m²·h. The amount of color change ΔE*ab is more preferably 1.2 or less, still more preferably 0.6 or less.

The amount of color change ΔE*ab of the color patches 12a to 12g is more preferably 2.5 or less after irradiation with light from a white LED light source having a color rendering of Ra ≥ 90 and R9 ≥ 80 and having a spectral distribution simulating that of standard illuminant D65 is performed at an irradiance of 50 W/m² to 65 W/m² on an irradiated object and a cumulative light dose of 1000 W/m²·h.

The amount of color change ΔE*ab of the color patches 12a to 12g is still more preferably 2.5 or less after irradiation with light from a white LED light source having a color rendering of Ra ≥ 90 and R9 ≥ 80 and having a spectral distribution simulating that of standard illuminant D65 is performed at an irradiance of 50 W/m² to 65 W/m² on an irradiated object and a cumulative light dose of 2000 W/m²·h.

The amount of color change ΔE*ab is a value determined from the color-difference formula (Equation B below) in the CIE 1976 (L*, a*, b*) color space system. The values of L*, a*, and b* are calculated by converting X, Y, and Z using Equation B below. The values of X, Y, and Z are calculated using Equation A above from the spectrum, color-matching functions, and relative spectral distribution of the illumination light. Ra represents the average color rendering index, and R9 represents the special color rendering index. ΔE*ab may also be simply referred to as ΔE.

ΔE*ab = 2.5 or less is known as a color difference in which "colors can be recognized as almost identical when assessed at a distance." ("New Edition Color Science Handbook [Third Edition]", The Color Science Association of Japan (The University of Tokyo Press)). When ΔE*ab is 2.5 or less, a high color-correction effect can be maintained.

ΔE*ab = 1.2 or less is known as a color difference that "can be readily recognized by most people when colors are assessed side by side." ("New Edition Color Science Handbook [Third Edition]", The Color Science Association of Japan (The University of Tokyo Press)). When ΔE*ab is 1.2 or less, a higher color-correction effect can be maintained.

ΔE*ab = 0.6 or less is known as a color difference that "corresponds to the practical tolerance limit when various error factors are taken into account." ("New Edition Color Science Handbook [Third Edition]", The Color Science Association of Japan (The University of Tokyo Press)). When ΔE*ab is 0.6 or less, an even higher color-correction effect can be maintained.

Thus, the color correction chart 10 according to this embodiment can maintain its high color-correction effect even after long-term use or multiple uses.

In the color correction chart 10 according to this embodiment, the change in transmittance at 500 nm in the color spectra of the color patches 12a to 12g after long-term light irradiation is reduced. More specifically, at least one of the amount of change in transmittance (%T) at 500 nm and the amount of change in transmittance (%T) at 520 nm in the color spectra of the color patches 12a to 12g is preferably 2.0 %T or less after irradiation with light from a white LED light source having a color rendering of Ra ≥ 90 and R9 ≥ 80 and having a spectral distribution simulating that of standard illuminant D65 is performed at an irradiance of 50 W/m² to 65 W/m² on an irradiated object and a cumulative light dose of 435 W/m²·h. The amount of change in at least one of these transmittances (%T) is more preferably 1.5 %T or less, still more preferably 1.0 %T or less.

The amount of change in transmittance at 500 nm in the spectra can be calculated from the following formula. Amount of change (%T) = transmittance after light irradiation (%T) - transmittance before light irradiation (%T)

At least one of the rate of change in transmittance (%T) at 500 nm and the rate of change in transmittance (%T) at 520 nm in the color spectra of the color patches 12a to 12g is preferably 10% or less after irradiation with light from a white LED light source having a color rendering of Ra ≥ 90 and R9 ≥ 80 and having a spectral distribution simulating that of standard illuminant D65 is performed at an irradiance of 50 W/m² to 65 W/m² on an irradiated object and a cumulative light dose of 435 W/m²·h. The rate of change in at least one of these transmittances (%T) is more preferably 8.0% or less, still more preferably 5.0% or less.

The rate of change (%) in transmittance at 500 nm in the spectrum can be calculated from the following formula.

Rate of change (%) = (transmittance after light irradiation (%T)/transmittance before light irradiation (%T)) - 1] × 100

The color correction chart 10 according to this embodiment may further include, in addition to the above components, an alignment mark, a recognition code, a cover glass, a color patch holding frame, and a transparent protective plate with a light-shielding portion. The recognition code may be a code in which information, such as test chart information, is recorded. The alignment mark may be a mark in which position information is recorded, and may also function as a recognition code in which information, such as test chart information, is recorded. These may be provided on the transparent protective plate with the light-shielding portion.

(Configuration of Each Color Patch)

The configuration of each color patch according to this embodiment will be described below.

(a) First Color Patch

The first color patch 12a contains a first color. The first color may be the most characteristic color for representing the color of a stained pathological cell preparation. Referring to Figs. 2A to 2C, the coordinate point of the first color is located within a region bounded by three points (0.200, 0.070), (0.450, 0.280), and (0.310, 0.340) on the xy chromaticity diagram. In other words, the coordinate point of the first color is located in the first color gamut L1 bounded by the triangle formed by straight lines connecting the coordinates (0.200, 0.070), (0.450, 0.280), and (0.310, 0.340) on the xy chromaticity diagram. The first color may be located within a coordinate range on the xy chromaticity diagram, but does not have to be within a predetermined white region. The predetermined white region may be, for example, the region defined by a blackbody locus of 5000 K or higher and 9300 K or lower and a color deviation of $d_{UV} = \pm 0.005$ from the blackbody locus.

Referring to Figs. 2A to 2C, the coordinate point of the first color is preferably located within a region bounded by five points (0.310, 0.340), (0.350, 0.300), (0.350, 0.200), (0.260, 0.150), and (0.280, 0.250) on the xy chromaticity diagram. In other words, the coordinate point of the first color is preferably located in the second color gamut L2 bounded by the pentagon formed by straight lines connecting the coordinates (0.310, 0.340), (0.350, 0.300), (0.350, 0.200), (0.260, 0.150), and (0.280, 0.250) on the xy chromaticity diagram in this order.

Referring to Figs. 2A to 2C, the coordinate point of the first color is more preferably located within a region bounded by four points (0.311, 0.334), (0.347, 0.210), (0.270, 0.162), and (0.292, 0.216) on the xy chromaticity diagram. In other words, the coordinate point of the first color is preferably located in the third color gamut L3 bounded by the rectangle formed by straight lines connecting the coordinates (0.311, 0.334), (0.347, 0.210), (0.270, 0.162), and (0.292, 0.216) on the xy chromaticity diagram in this order.

In an example, the coordinate point of the first color may be, for example, the coordinates (0.328, 0.224) on the xy chromaticity diagram. The coordinate point of the first color may lie at a vertex of any of the first to third color gamuts L1 to L3 on the xy chromaticity diagram, or may lie on a straight line connecting vertices of the first to third color gamuts L1 to L3.

Referring to Fig, 4, the peak wavelength $\lambda P$ of the spectrum of the first color may be 520 nm or more and 560 nm or less. The spectral transmittance TP at the peak wavelength $\lambda P$ of the spectrum of the first color may be 0.5 %T or more, may be 1 %T or more, may be 3 %T or more, or may be 5 %T or more. The spectral transmittance TP at the peak wavelength $\lambda P$ may be 20 %T or less, may be 15 %T or less, or may be 10 %T or less.

The first color may be the most characteristic color for representing the color of a stained pathological cell preparation, as described above. The first color may be obtained, for example, by acquiring the spectra of stained cell preparations from various organs and selecting and extracting characteristic cell colors from the acquired spectra. In this case, the first color may be selected, for example, using a statistical method on the basis of the extracted spectra of the stained cell preparations.

(b) Second Color Patch

The second color patch 12b contains a second color different from the first color. The second color may be a characteristic color for representing the color of a stained pathological cell preparation. Referring to Figs. 2A to 2C, the coordinate point of the second color is located in the first color gamut L1 bounded by three points (0.200, 0.070), (0.450, 0.280), and (0.310, 0.340) on the xy chromaticity diagram. The second color may be located within a coordinate range on the xy chromaticity diagram, but does not have to be within a predetermined white region. The predetermined white region may be, for example, the region defined by a blackbody locus of 5000 K or higher and 9300 K or lower and a color deviation of $d_{UV} = \pm 0.005$ from the blackbody locus. In this description, the term "different color" includes not only a color located at different coordinates on the xy chromaticity diagram, but also a color located at the same coordinates on the xy chromaticity diagram but having different lightness or a different spectrum.

Referring to Figs. 2A to 2C, the coordinate point of the second color is preferably located in the second color gamut L2 bounded by five points (0.310, 0.340), (0.350, 0.300), (0.350, 0.200), (0.260, 0.150), and (0.280, 0.250) on the xy chromaticity diagram.

Referring to Figs. 2A to 2C, the coordinate point of the second color is preferably located in the third color gamut L3 bounded by four points (0.311, 0.334), (0.347, 0.210), (0.270, 0.162), and (0.292, 0.216) on the xy chromaticity diagram.

In an example, the coordinate point of the second color may be, for example, the coordinates (0.275, 0.172) on the xy chromaticity diagram. The coordinate point of the second color may lie at a vertex of any of the first to third color gamuts L1 to L3 on the xy chromaticity diagram, or may lie on a straight line connecting vertices of the first to third color gamuts L1 to L3.

Referring to Fig, 4, the peak wavelength $\lambda P$ of the spectrum of the second color may be 520 nm or more and 560 nm or less. The spectral transmittance TP at the peak wavelength $\lambda P$ of the spectrum of the second color may be 0.5 %T or more, may be 1 %T or more, or may be 2 %T or more. The spectral transmittance TP at the peak wavelength $\lambda P$ may be 10 %T or less, may be 8 %T or less, or may be 5 %T or less. The spectral transmittance TP at the peak wavelength $\lambda P$ of the spectrum of the second color may be lower than the spectral transmittance TP at the peak wavelength $\lambda P$ of the spectrum of the first color.

The second color may be a characteristic color for representing the color of a stained pathological cell preparation, as described above. The second color may be located within the first color gamut L1 on the xy chromaticity diagram and at a distance greater than or equal to a predetermined distance from the first color on the xy chromaticity diagram. In this case, the color of the pathological specimen image is easily corrected.

(c) Third Color Patch

The third color patch 12c contains a third color different from the first color and the second color. The third color may be a characteristic color for representing the color of a stained pathological cell preparation. Referring to Figs. 2A to 2C, the coordinate point of the third color is located in the first color gamut L1 bounded by three points (0.200, 0.070), (0.450, 0.280), and (0.310, 0.340) on the xy chromaticity diagram. The third color may be located within a coordinate range on the xy chromaticity diagram, but does not have to be within a predetermined white region. The predetermined white region may be, for example, the region defined by a blackbody locus of 5000 K or higher and 9300 K or lower and a color deviation of $d_{UV} = \pm 0.005$ from the blackbody locus.

Referring to Figs. 2A to 2C, the coordinate point of the third color is preferably located in the second color gamut L2 bounded by five points (0.310, 0.340), (0.350, 0.300), (0.350, 0.200), (0.260, 0.150), and (0.280, 0.250) on the xy chromaticity diagram.

Referring to Figs. 2A to 2C, the coordinate point of the third color is preferably located in the third color gamut L3 bounded by four points (0.311, 0.334), (0.347, 0.210), (0.270, 0.162), and (0.292, 0.216) on the xy chromaticity diagram.

In an example, the coordinate point of the third color may be, for example, the coordinates (0.327, 0.270) on the xy chromaticity diagram. The coordinate point of the third color may lie at a vertex of any of the first to third color gamuts L1 to L3 on the xy chromaticity diagram, or may lie on a straight line connecting vertices of the first to third color gamuts L1 to L3.

Referring to Fig, 4, the peak wavelength $\lambda P$ of the spectrum of the third color may be 520 nm or more and 560 nm or less. The spectral transmittance TP at the peak wavelength $\lambda P$ of the spectrum of the third color may be 10 %T or more, may be 15 %T or more, or may be 20 %T or more. The spectral transmittance TP at the peak wavelength $\lambda P$ may be 45 %T or less, may be 40 %T or less, or may be 35 %T or less. The spectral transmittance TP at the peak wavelength $\lambda P$ of the spectrum of the third color may be higher than the spectral transmittance TP at the peak wavelength $\lambda P$ of the spectrum of the first color.

The third color may be a characteristic color for representing the color of a stained pathological cell preparation, as described above. The third color may be located within the first color gamut L1 on the xy chromaticity diagram and at a distance greater than or equal to a predetermined distance from the first color and the second color on the xy chromaticity diagram. In this case, the color of the pathological specimen image is easily corrected.

(d) Fourth Color Patch

The fourth color patch 12d contains a fourth color different from the first to third colors. The fourth color may be a characteristic color for representing the color of a stained pathological cell preparation. Referring to Figs. 2A to 2C, the coordinate point of the fourth color is located in the first color gamut L1 bounded by three points (0.200, 0.070), (0.450, 0.280), and (0.310, 0.340) on the xy chromaticity diagram. The fourth color may be located within a coordinate range on the xy chromaticity diagram, but does not have to be within a predetermined white region. The predetermined white region may be, for example, the region defined by a blackbody locus of 5000 K or higher and 9300 K or lower and a color deviation of $d_{UV} = \pm 0.005$ from the blackbody locus.

Referring to Figs. 2A to 2C, the coordinate point of the fourth color is preferably located in the second color gamut L2 bounded by five points (0.310, 0.340), (0.350, 0.300), (0.350, 0.200), (0.260, 0.150), and (0.280, 0.250) on the xy chromaticity diagram.

Referring to Figs. 2A to 2C, the coordinate point of the fourth color is preferably located in the third color gamut L3 bounded by four points (0.311, 0.334), (0.347, 0.210), (0.270, 0.162), and (0.292, 0.216) on the xy chromaticity diagram.

In an example, the coordinate point of the fourth color may be, for example, the coordinates (0.321, 0.239) on the xy chromaticity diagram. The coordinate point of the fourth color may lie at a vertex of any of the first to third color gamuts L1 to L3 on the xy chromaticity diagram, or may lie on a straight line connecting vertices of the first to third color gamuts L1 to L3.

Referring to Fig, 4, the peak wavelength $\lambda P$ of the spectrum of the fourth color may be 520 nm or more and 560 nm or less. The spectral transmittance TP at the peak wavelength $\lambda$P of the spectrum of the fourth color may be 3 %T or more, may be 5 %T or more, or may be 7 %T or more. The spectral transmittance TP at the peak wavelength $\lambda$P may be 25 %T or less, may be 20 %T or less, or may be 15 %T or less. The spectral transmittance TP at the peak wavelength $\lambda$P of the spectrum of the fourth color may be higher than the spectral transmittance TP at the peak wavelength $\lambda$P of the spectrum of the first color.

The fourth color may be a characteristic color for representing the color of a stained pathological cell preparation, as described above. The fourth color may be located within the first color gamut L1 on the xy chromaticity diagram and at a distance greater than or equal to a predetermined distance from at least the first color on the xy chromaticity diagram. In this case, the color of the pathological specimen image is easily corrected.

(e) Fifth Color Patch

The fifth color patch 12e contains a fifth color different from the first to fourth colors. The fifth color may be a characteristic color for representing the color of a stained pathological cell preparation. Referring to Figs. 2A to 2C, the coordinate point of the fifth color is located in the first color gamut L1 bounded by three points (0.200, 0.070), (0.450, 0.280), and (0.310, 0.340) on the xy chromaticity diagram. The fifth color may be located within a coordinate range on the xy chromaticity diagram, but does not have to be within a predetermined white region. The predetermined white region may be, for example, the region defined by a blackbody locus of 5000 K or higher and 9300 K or lower and a color deviation of $d_{UV} = \pm 0.005$ from the blackbody locus.

Referring to Figs. 2A to 2C, the coordinate point of the fifth color is preferably located in the second color gamut L2 bounded by five points (0.310, 0.340), (0.350, 0.300), (0.350, 0.200), (0.260, 0.150), and (0.280, 0.250) on the xy chromaticity diagram.

Referring to Figs. 2A to 2C, the coordinate point of the fifth color is preferably located in the third color gamut L3 bounded by four points (0.311, 0.334), (0.347, 0.210), (0.270, 0.162), and (0.292, 0.216) on the xy chromaticity diagram.

In an example, the coordinate point of the fifth color may be, for example, the coordinates (0.320, 0.195) on the xy chromaticity diagram. The coordinate point of the fifth color may lie at a vertex of any of the first to third color gamuts L1 to L3 on the xy chromaticity diagram, or may lie on a straight line connecting vertices of the first to third color gamuts L1 to L3.

Referring to Fig, 4, the peak wavelength $\lambda$P of the spectrum of the fifth color may be 520 nm or more and 560 nm or less. The spectral transmittance TP at the peak wavelength $\lambda$P of the spectrum of the fifth color may be 0.5 %T or more, may be 1 %T or more, may be 2 %T or more, or may be 3 %T or more. The spectral transmittance TP at the peak wavelength $\lambda$P may be 15 %T or less, may be 10 %T or less, or may be 7 %T or less. The spectral transmittance TP at the peak wavelength $\lambda$P of the spectrum of the fifth color may be lower than the spectral transmittance TP at the peak wavelength $\lambda$P of the spectrum of the first color.

The fifth color may be a characteristic color for representing the color of a stained pathological cell preparation, as described above. The fifth color may be located within the first color gamut L1 on the xy chromaticity diagram and at a distance greater than or equal to a predetermined distance from at least the first color on the xy chromaticity diagram. In this case, the color of the pathological specimen image is easily corrected.

(f) Sixth Color Patch

The sixth color patch 12f contains a sixth color different from the first to fifth colors. The sixth color may be a characteristic color for representing the color of a stained pathological cell preparation. Referring to Figs. 2A to 2C, the coordinate point of the sixth color is located in the first color gamut L1 bounded by three points (0.200, 0.070), (0.450, 0.280), and (0.310, 0.340) on the xy chromaticity diagram.

Referring to Figs. 2A to 2C, the coordinate point of the sixth color is preferably located in the second color gamut L2 bounded by five points (0.310, 0.340), (0.350, 0.300), (0.350, 0.200), (0.260, 0.150), and (0.280, 0.250) on the xy chromaticity diagram. The sixth color may be located within a coordinate range on the xy chromaticity diagram, but does not have to be within a predetermined white region. The predetermined white region may be, for example, the region defined by a blackbody locus of 5000 K or higher and 9300 K or lower and a color deviation of $d_{UV} = \pm 0.005$ from the blackbody locus.

Referring to Figs. 2A to 2C, the coordinate point of the sixth color is preferably located in the third color gamut L3 bounded by four points (0.311, 0.334), (0.347, 0.210), (0.270, 0.162), and (0.292, 0.216) on the xy chromaticity diagram.

In an example, the coordinate point of the sixth color may be, for example, the coordinates (0.331, 0.213) on the xy chromaticity diagram. The coordinate point of the sixth color may lie at a vertex of any of the first to third color gamuts L1 to L3 on the xy chromaticity diagram, or may lie on a straight line connecting vertices of the first to third color gamuts L1 to L3.

Referring to Fig, 4, the peak wavelength $\lambda$P of the spectrum of the sixth color may be 520 nm or more and 560 nm or less. The spectral transmittance TP at the peak wavelength $\lambda$P of the spectrum of the sixth color may be 1 %T or more, may be 2 %T or more, or may be 3 %T or more. The spectral transmittance TP at the peak wavelength $\lambda$P may be 15 %T or less, may be 10 %T or less, or may be 7 %T or less. The spectral transmittance TP at the peak wavelength $\lambda$P of the spectrum of the sixth color may be lower than the spectral transmittance TP at the peak wavelength $\lambda$P of the spectrum of the first color.

The sixth color may be a characteristic color for representing the color of a stained pathological cell preparation, as described above. The sixth color may be located within the first color gamut L1 on the xy chromaticity diagram and at a distance greater than or equal to a predetermined distance from at least the first color on the xy chromaticity diagram. In this case, the color of the pathological specimen image is easily corrected.

(g) Seventh Color Patch

The seventh color patch 12g contains a seventh color different from the first to sixth colors. The seventh color may be a characteristic color for representing the color of a stained pathological cell preparation. Referring to Figs. 2A to 2C, the coordinate point of the seventh color is located in the first color gamut L1 bounded by three points (0.200, 0.070), (0.450, 0.280), and (0.310, 0.340) on the xy chromaticity diagram. The seventh color may be located within a coordinate range on the xy chromaticity diagram, but does not have to be within a predetermined white region. The predetermined white region may be, for example, the region defined by a blackbody locus of 5000 K or higher and 9300 K or lower and a color deviation of $d_{UV}$ = $\pm$0.005 from the blackbody locus.

Referring to Figs. 2A to 2C, the coordinate point of the seventh color is preferably located in the second color gamut L2 bounded by five points (0.310, 0.340), (0.350, 0.300), (0.350, 0.200), (0.260, 0.150), and (0.280, 0.250) on the xy chromaticity diagram.

Referring to Figs. 2A to 2C, the coordinate point of the seventh color is preferably located in the third color gamut L3 bounded by four points (0.311, 0.334), (0.347, 0.210), (0.270, 0.162), and (0.292, 0.216) on the xy chromaticity diagram.

In an example, the coordinate point of the seventh color may be, for example, the coordinates (0.309, 0.223) on the xy chromaticity diagram. The coordinate point of the seventh color may lie at a vertex of any of the first to third color gamuts L1 to L3 on the xy chromaticity diagram, or may lie on a straight line connecting vertices of the first to third color gamuts L1 to L3.

Referring to Fig, 4, the peak wavelength $\lambda$P of the spectrum of the seventh color may be 520 nm or more and 560 nm or less. The spectral transmittance TP at the peak wavelength $\lambda$P of the spectrum of the seventh color may be 1 %T or more, may be 3 %T or more, or may be 5 %T or more. The spectral transmittance TP at the peak wavelength $\lambda$P may be 25 %T or less, may be 20 %T or less, or may be 15 %T or less. The spectral transmittance TP at the peak wavelength $\lambda$P of the spectrum of the seventh color may be higher than the spectral transmittance TP at the peak wavelength $\lambda$P of the spectrum of the first color.

The seventh color may be a characteristic color for representing the color of a stained pathological cell preparation, as described above. The seventh color may be located within the first color gamut L1 on the xy chromaticity diagram and at a distance greater than or equal to a predetermined distance from at least the first color on the xy chromaticity diagram. In this case, the color of the pathological specimen image is easily corrected.

(h) Additional Color Patch

The color patch group 12 may have an additional color patch other than the color patches 12a to 12g. The additional color patch may contain a color different from the first to seventh colors. Referring to Figs. 3A to 3C, the color coordinate point of the additional color patch may be located in the first color gamut L1 bounded by three points (0.200, 0.070), (0.450, 0.280), and (0.310, 0.340) on the xy chromaticity diagram. The color of the additional color patch may be located within a coordinate range on the xy chromaticity diagram, but does not have to be within a predetermined white region. The predetermined white region may be, for example, the region defined by a blackbody locus of 5000 K or higher and 9300 K or lower and a color deviation of $d_{UV}$ = $\pm$0.005 from the blackbody locus.

The additional color patch may have a color similar to that of red blood cells. Referring to Figs. 3A to 3C, the color of the additional color patch may be located within a region bounded by three points (0.350, 0.300), (0.350, 0.200), and (0.380, 0.280) on the xy chromaticity diagram. In other words, the color coordinate point of the additional color patch may be included in the fourth color gamut L4 on the xy chromaticity diagram.

In an example, the color coordinate point of the additional color patch may be, for example, the coordinates (0.368, 0.278) on the xy chromaticity diagram. The color coordinate point of the additional color patch may be at any of the vertices of the fourth color gamut L4 on the xy chromaticity diagram, or may be on a straight line connecting the vertices of the fourth color gamut L4.

Referring to Figs. 3A to 3C, at least one of the colors of the color patches 12a to 12g may be located within a region bounded by three points (0.350, 0.300), (0.350, 0.200), and (0.380, 0.280) on the xy chromaticity diagram. In other words, the coordinate point of at least one of the colors of the color patches 12a to 12g may be included in the fourth color gamut L4 on the xy chromaticity diagram.

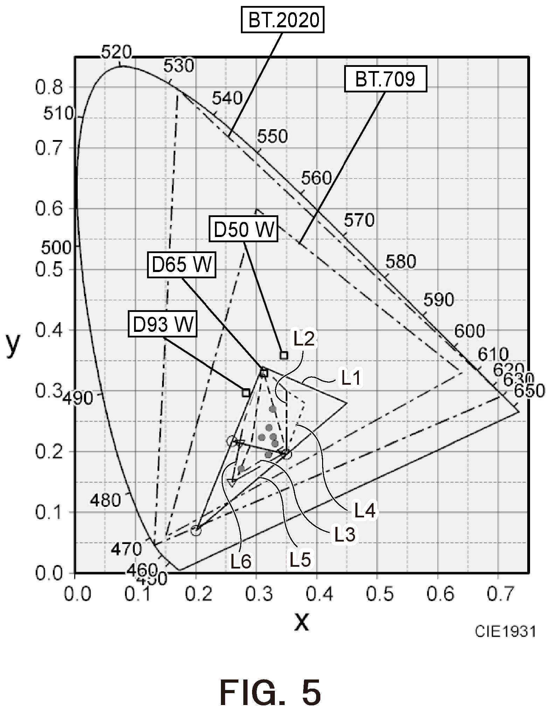

Referring to Fig. 5, at least one of the colors of the color patches 12a to 12g may be located within a region bounded by three points (0.200, 0.070), (0.260, 0.217), and (0.350, 0.196) on the xy chromaticity diagram. In other words, the color coordinate points of the color patches 12a to 12g may be located in a fifth color gamut L5 bounded by a triangle formed by straight lines connecting the coordinates (0.200, 0.070), (0.260, 0.217), and (0.350, 0.196) on the xy chromaticity diagram. When the color coordinate points of the color patches 12a to 12g are located in the fifth color gamut L5 on the xy chromaticity diagram, the color of nuclei can be accurately corrected.

Referring to Fig. 5, at least one of the colors of the color patches 12a to 12g may be located within a region bounded by three points (0.273, 0.214), (0.345, 0.197), and (0.260, 0.150) on the xy chromaticity diagram. In other words, the color coordinate points of the color patches 12a to 12g may be located in a sixth color gamut L6 bounded by a triangle formed by straight lines connecting the coordinates (0.273, 0.214), (0.345, 0.197), and (0.260, 0.150) on the xy chromaticity diagram. When the color coordinate points of the color patches 12a to 12g are located in the sixth color gamut L6 on the xy chromaticity diagram, the color of nuclei can be accurately corrected.

The color patch group 12 may have standard color patches other than the color patches 12a to 12g. Examples of the standard color patches include colors defined in the BT.709 standard or colors defined in the BT.2020 standard. The standard color patches may include, for example, a red (R) color patch, a green (G) color patch, a blue (B) color patch, a magenta (M) color patch, a yellow (Y) color patch, a cyan (C) color patch, and a violet (V) color patch.

The colors of the standard color patches may be, for example, colors within the coordinate ranges referred to as blue, red, green, blue-green, purple to red-purple, pink (purplish pink), and yellow in the figure (general chromaticity classification of system color names) shown in JIS Z 8110. The colors of the standard color patches may be colors corresponding to Red, Green, Blue, Cyan, Magenta, and Yellow in commercially available color charts. Examples of commercially available color charts include ColorChecker (X-Rite, Inc. (formerly GretagMacbeth)), IT8 Target (FUJIFILM Corporation, Eastman Kodak Company, and LaserSoft Imaging AG), BT.709 charts, and BT.2020 charts. The color representations used for web colors may be colors referred to as Red for R, Green for G, Blue for B, Cyan or Aqua for C, Magenta or Fuchsia for M, and Yellow for Y.

The color patch group 12 may include grayscale color patches for luminance control. The grayscale color patches may be color patches composed of colors having different luminance levels, such as black, gray, and white.

The color patch group 12 including the standard color patches or the grayscale color patches facilitates color correction of an imaging device even if a portion of a stained pathological cell preparation exhibits a color that does not fall within the first color gamut L1 on the xy chromaticity diagram.

(Method for Forming Color Patches)

The color patches 12a to 12g for the respective colors are members that each exhibit a desired transmission spectrum. The color patches 12a to 12g for the respective colors may be, for example, band-pass filters or dyed substrates. The dyed substrates can be formed by a dyeing method. The dyed substrates can be produced, for example, as follows. First, a silver halide emulsion is applied onto a chip substrate, such as a glass plate. The silver halide emulsion may be prepared by adding potassium bromide and a silver nitrate solution to gelatin. Next, silver is removed from a silver halide photographic dry plate obtained by drying the silver halide emulsion. The silver halide photographic dry plate is then dyed with a dye corresponding to the color of a color patch to form a dyed substrate. The dyed substrate may be produced as follows. First, gelatin (solution) is previously mixed with the dye to prepare a material having a predetermined color. This material may be applied onto a chip substrate, such as a glass plate, to form a dyed substrate.

The colors of the color patches 12a to 12g may be obtained by acquiring the actual spectra of stained cell preparations from various organs and selecting and extracting characteristic cell colors from the acquired spectra. In that case, the colors of the color patches 12a to 12g may be selected, for example, using a statistical method on the basis of the extracted spectra of the stained cell preparations.

In forming the color patches 12a to 12g, the colors and transmission spectra of the color patches 12a to 12g can be adjusted according to the types of color patches 12a to 12g and their formation methods. For example, when dyed substrates formed with a single dye are used as the color patches 12a to 12g, the colors and transmission spectra of the color patches 12a to 12g can be adjusted by varying the concentration of the dye. When dyed substrates formed with a mixture of two or more dyes are used as the color patches 12a to 12g, the colors and transmission spectra of the color patches 12a to 12g can be adjusted by varying the mixing ratio of two dyes.

The size and other parameters of the color patches 12a to 12g are not limited and can be appropriately designed so as to achieve a desired effect according to, for example, the intended use of the color correction chart 10 of this embodiment.

In the color patch group 12, the color patches 12a to 12g are arranged in a pattern. With regard to the arrangement pattern of the color patches 12a to 12g, the color patches may be arranged in a line pattern in a single row, as illustrated in Fig. 1. Alternatively, the color patches 12a to 12g may be arranged in a grid pattern or a circular pattern, although not illustrated. The order of arrangement of the color patches 12a to 12g is not limited. The arrangement of the color patches 12a to 12g can be appropriately designed.

Each of the color patches 12a to 12g can also be formed by a conventionally known method, such as a vapor deposition method, a dyeing method, a printing method, a transfer method, or an inkjet method. The method for forming the color patches 12a to 12g by the dyeing method is described above, and therefore, the description thereof is omitted here. The color patch group 12 may be formed, for example, by arranging the color patches 12a to 12g, which are formed by the above method, in a desired pattern on one surface of the substrate 11, and sandwiching the color patches 12a to 12g between the substrate 11 and a cover glass.

The size of the color correction chart 10 according to this embodiment can be designed in accordance with the target captured image. For example, the color correction chart 10 according to this embodiment can be used for color evaluation and color correction of an output image of a measurement sample captured with a microscope of a pathological imaging device. In this case, the color correction chart 10 may be a micro-imaging color chart having the color patch group 12 with a size corresponding to the magnification of the objective lens of the microscope. The color correction chart 10 according to this embodiment can also be used, for example, for color evaluation and color correction of an output image of a measurement sample captured at 1:1 magnification by an imaging device. In this case, the color correction chart 10 may be a micro-imaging color chart having the color patch group 12 with a size corresponding to the size of the captured image.

The color correction chart 10 according to this embodiment can be used for pathological imaging devices and all peripheral devices that require color correction of stained pathological cell preparations. In this description, the term "imaging device" includes not only imaging devices that exclusively capture still images but also video imaging devices that capture moving images and the like. For example, a WSI (whole slide imaging) scanner may be used as a pathological imaging device.

Next, a method for color correction of a stained pathological cell preparation using the color correction chart 10 according to this embodiment will be described.

First, a stained pathological cell preparation is prepared. In this case, pathological cells are first collected as a specimen and subjected to a pretreatment, such as degreasing. Next, the pathological cells are sliced into thin sections and stained with a staining solution, such as hematoxylin and eosin (HE). Hematoxylin solution, which is blue-purple in color, stains cell nuclei and ribosomes blue-purple. Eosin solution, which is red in color, stains cytoplasm, intercellular connective tissues, fibers, red blood cells, acidophilic substances, and granules red or purple. As a result, the cell nuclei, cytoplasm, osseous tissue, connective tissue of soft tissues, red blood cells, fibrous tissue, endocrine granules, extracellular matrix, and the like contained in the pathological cells are stained.

Next, an image of the stained pathological cell preparation thus obtained is captured using an imaging device, such as a microscope or a glass-slide scanner. In this step, the image of the stained pathological cell preparation may be captured together with the color correction chart 10 according to this embodiment so that both are included within the imaging area of the imaging device. Alternatively, the image of the color correction chart 10 according to this embodiment may be obtained separately. The obtained image is a digital image.

Next, color correction is performed on the digital image using an image correction program. In this case, an ICC (International Color Consortium) profile is first generated on the basis on the captured image of the color correction chart 10 by applying the image correction program. The term "ICC profile" refers to information that defines color correction and the like performed in a device that is the subject of color correction. Thereafter, the ICC profile is applied to the image of the stained pathological cell preparation to perform color correction on the image of the stained pathological cell preparation.

As described above, according to this embodiment, the color coordinate points of the color patches 12a to 12g are located within a region bounded by three points (0.200, 0.070), (0.450, 0.280), and (0.310, 0.340) on the xy chromaticity diagram. This can make the colors of the color patches 12a to 12g closer to those of actual HE-stained pathological cell preparations. As a result, color correction of an imaging device using the color correction chart 10 can be more easily performed, and color correction can be effectively performed on an imaging device.

According to this embodiment, the color coordinate points of the color patches 12a to 12g may be located within a region bounded by five points (0.310, 0.340), (0.350, 0.300), (0.350, 0.200), (0.260, 0.150), and (0.280, 0.250) on the xy chromaticity diagram. Accordingly, color correction can be more effectively performed on an imaging device using the color correction chart 10.

According to this embodiment, the color coordinate points of the color patches 12a to 12g are located within a region bounded by four points (0.311, 0.334), (0.347, 0.210), (0.270, 0.162), and (0.292, 0.216) on the xy chromaticity diagram. Accordingly, color correction can be more effectively performed on an imaging device using the color correction chart 10.

EXAMPLES

Next, specific Examples in this embodiment will be described.

EXAMPLE 1

A color correction chart (Example 1) was prepared. This color correction chart (Example 1) had seven color patches of the first to seventh colors. In the color correction chart (Example 1), the color coordinates of the first to seventh colors on the xy chromaticity diagram are as follows. All of the first to seventh colors are located within a region bounded by four points (0.311, 0.334), (0.347, 0.210), (0.270, 0.162), and (0.292, 0.216) on the xy chromaticity diagram. In other words, all of the first to seventh colors are located within the first color gamut L1, the second color gamut L2, and the third color gamut L3 on the xy chromaticity diagram.

[Table 1]

| Color Patch | x | y |
|---|---|---|
| First color | 0.328 | 0.224 |
| Second color | 0.275 | 0.172 |
| Third color | 0.327 | 0.270 |
| Fourth color | 0.321 | 0.239 |
| Fifth color | 0.320 | 0.195 |
| Sixth color | 0.331 | 0.213 |
| Seventh color | 0.309 | 0.223 |

The color correction chart (Example 1) also had color patches of the standard colors defined in the BT.709 standard, and grayscale color patches. The standard color patches included seven color patches: a red (R) color patch, a green (G) color patch, a blue (B) color patch, a magenta (M) color patch, a yellow (Y) color patch, a cyan (C) color patch, and a violet (V) color patch. The grayscale color patches had 11 gradations from black to white.

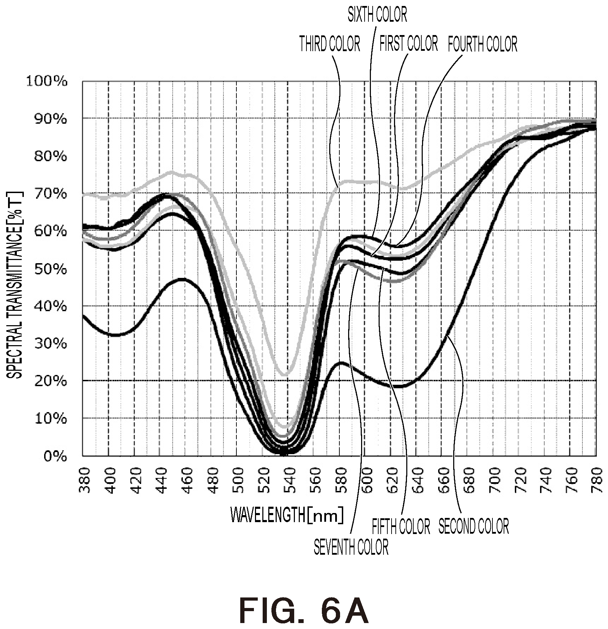

Fig. 6A illustrates the spectra of the color patches of the first to seventh colors of the color correction chart. In the spectra illustrated in Fig. 6A, the peak wavelength of the spectrum of each color patch was in the range of 520 nm or more and 560 nm or less, and the spectral transmittance at the peak wavelength was 0.5 %T or more and 30 %T or less.

EXAMPLE 2

A color correction chart (Example 2) was prepared. This color correction chart had four color patches of the eighth to eleventh colors. In the color correction chart, the color coordinates of the eighth to eleventh colors on the xy chromaticity diagram are as follows. All of the eighth to eleventh colors are located within a region bounded by five points (0.310, 0.340), (0.350, 0.300), (0.350, 0.200), (0.260, 0.150), and (0.280, 0.250) on the xy chromaticity diagram. None of the eighth to eleventh colors is located within a region bounded by four points (0.311, 0.334), (0.347, 0.210), (0.270, 0.162), and (0.292, 0.216) on the xy chromaticity diagram. In other words, all of the eighth to eleventh colors are located within the first color gamut L1 and the second color gamut L2, and none of them is located within the third color gamut L3 on the xy chromaticity diagram.

[Table 2]

| Color Patch | x | y |
|---|---|---|
| Eighth color | 0.332 | 0.285 |
| Ninth color | 0.332 | 0.289 |
| Tenth color | 0.329 | 0.292 |
| Eleventh color | 0.286 | 0.236 |

The color correction chart (Example 2) also had color patches of the standard colors defined in the BT.709 standard, and grayscale color patches. The standard color patches and the grayscale color patches were identical to those used in the color correction chart (Example 1).

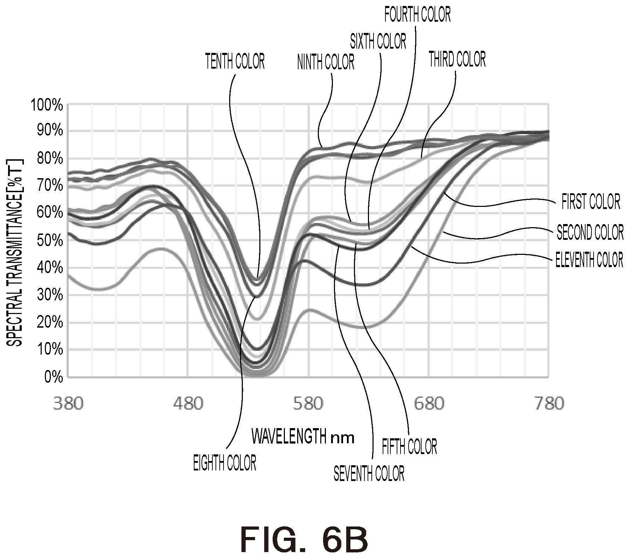

Fig. 6B illustrates the spectra of the color patches of the first to eleventh colors of the color correction chart. In the spectra illustrated in Fig. 6B, the peak wavelength of the spectrum of each color patch was in the range of 520 nm or more and 560 nm or less, and the spectral transmittance at the peak wavelength was 0.5 %T or more and 40 %T or less.

EXAMPLE 3

A color correction chart (Example 3) was prepared. This color correction chart had eleven color patches of the first to eleventh colors. In the color correction chart, the color coordinates of the first to seventh colors on the xy chromaticity diagram are identical to those in the color correction chart (Example 1). The color coordinates of the eighth to eleventh colors on the xy chromaticity diagram are identical to those in the color correction chart (Example 2).

The color correction chart (Example 3) also had color patches of the standard colors defined in the BT.709 standard, and grayscale color patches. The standard color patches and the grayscale color patches were identical to those used in the color correction chart (Example 1).

EXAMPLE 4

A color correction chart (Example 4) was prepared. This color correction chart had fourteen color patches of the first to fourteenth colors. In the color correction chart (Example 4), the color coordinates of the first to seventh colors on the xy chromaticity diagram are identical to those in the color correction chart (Example 1). The color coordinates of the eighth to eleventh colors on the xy chromaticity diagram are identical to those in the color correction chart (Example 2).

In the color correction chart, the color coordinates of the twelfth to fourteenth colors on the xy chromaticity diagram are as follows. Both the twelfth and fourteenth colors are located within the first color gamut L1 and the second color gamut L2, and none of them is located within the third color gamut L3 or the fourth color gamut L4 on the xy chromaticity diagram. The thirteenth color is located within a region bounded by three points (0.350, 0.300), (0.350, 0.200), and (0.380, 0.280) on the xy chromaticity diagram. In other words, the thirteenth color is located within the first color gamut L1 and the fourth color gamut L4, but not located within the second color gamut L2 or the third color gamut L3 on the xy chromaticity diagram.

[Table 3]

| Color Patch | x | y |
|---|---|---|
| Twelfth color | 0.281 | 0.202 |
| Thirteenth color | 0.368 | 0.278 |
| Fourteenth color | 0.338 | 0.248 |

The color correction chart (Example 4) had color patches of the standard colors defined in the BT.709 standard, and grayscale color patches. The standard color patches and the grayscale color patches were identical to those used in the color correction chart (Example 1).

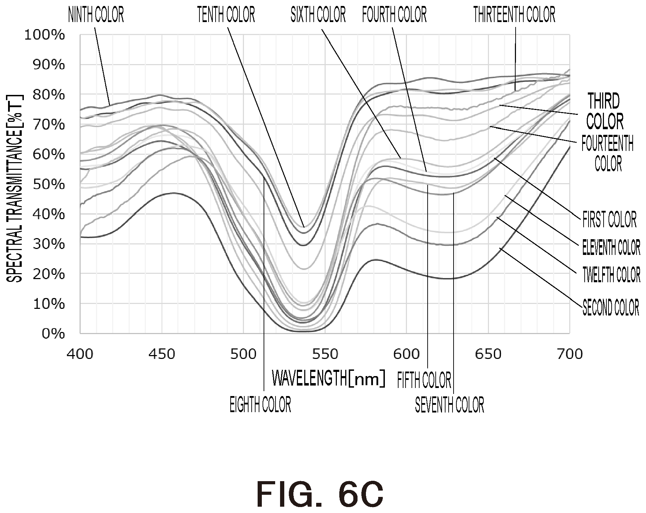

Fig. 6C illustrates the spectra of the color patches of the first to fourteenth colors of the color correction chart. In the spectra illustrated in Fig. 6C, the peak wavelength of the spectrum of each color patch was in the range of 520 nm or more and 560 nm or less, and the spectral transmittance at the peak wavelength was 0.5 %T or more and 40 %T or less.

COMPATATIVE EXAMPLE

A color correction chart (Comparative Example) was prepared in the same manner as in Example 1 above, except that the color correction chart did not have seven color patches of the first to seventh colors. This color correction chart had color patches of the standard colors defined in the BT.709 standard, and grayscale color patches. The standard color patches and the grayscale color patches were identical to those used in the color correction chart (Example 1).

[Investigation of Color Correction Accuracy for Pathological Images]

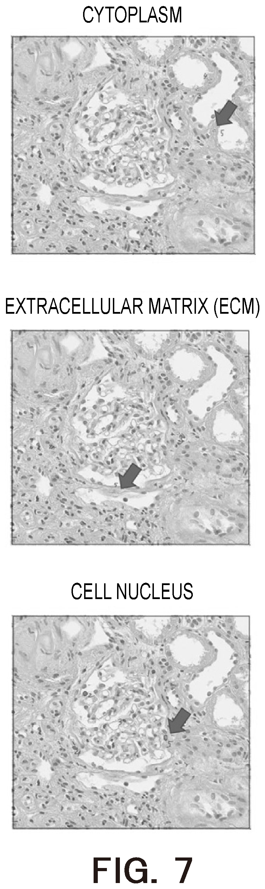

For each of the color correction charts (Examples 1 to 3 and Comparative Example), the accuracy of color correction for pathological images was investigated. In this case, a specimen of an actual HE-stained pathological cell preparation as illustrated in Fig. 7 was first prepared. Subsequently, the specimen of the stained pathological cell preparation was imaged using a WSI scanner (NanoZoomer HT1 available from Hamamatsu Photonics K.K.). Next, in the captured original image, five points each were extracted from cytoplasm, extracellular matrix (ECM), red blood cells (RBCs), and cell nuclei, and color correction was performed on the extracted points using each of the color correction charts (Examples 1 to 3 and Comparative Example).

Next, for (i) the captured original image and (ii) the images that have been color-corrected using the respective color correction charts (Examples 1 to 3 and Comparative Example), the color difference $\Delta E^*ab$ from the actual stained pathological cell preparation was measured.

The color difference ΔE*ab represents the difference between the L*a*b* values of a digital image and the measured corresponding L*a*b* values. The L*a*b* values of the digital image were obtained by converting the RGB values of the image acquired with a WSI scanner to XYZ values and then further converting the XYZ values to Lab values. The measured L*a*b* values were obtained from the spectrum of the actual stained pathological cell preparation using a spectroradiometer (SR-5000HM, available from Topcon Technohouse Corporation).

The color difference ΔE*ab is a value determined from the color-difference formula ($\Delta E^*ab = \{(\Delta L^*)^2 + (\Delta a^*)^2 + (\Delta b^*)^2\}^{1/2}$) in the CIE 1976 (L*, a*, b*) color space. The L*, a*, and b* are calculated by conversion from the tristimulus values X, Y, and Z using the following equations (collectively referred to as Equation B).

$$L^* = 116(Y/Yn)^{1/3} - 16$$

$$a^* = 500\{(X/Xn)^{1/3} - (Y/Yn)^{1/3}\}$$

$$b^* = 200\{(Y/Yn)^{1/3} - (Z/Zn)^{1/3}\}$$

In the equation, the function f(X/Xn) is defined as follows:

$$f(X/Xn) = (X/Xn)^{1/3} \quad (X/Xn > 0.008856)$$

$$f(X/Xn) = 7.787(X/Xn) + 16/116 \quad (X/Xn \leq 0.008856).$$

The functions f(Y/Yn) and f(Z/Zn) are defined similarly. In the above equations, Xn, Yn, and Zn are the tristimulus values of a perfect reflecting diffuser (or a perfect transmitting diffuser for a transmitting object). Xn, Yn, and Zn are calculated using the tristimulus values of a D65 illuminant ($\lambda$ = 380 nm to 780 nm, at 1-nm intervals). Xn = 95.04, Yn = 100.00, and Zn = 108.86 are used.

The above equations apply when X/Xn > 0.008856, Y/Yn > 0.008856, and Z/Zn > 0.008856; otherwise, the following correction equations apply.

$$L^* = 116f(Y/Yn) - 16$$

$$a^* = 500\{f(X/Xn) - f(Y/Yn)\}$$

$$b^* = 200\{f(Y/Yn) - f(Z/Zn)\}$$

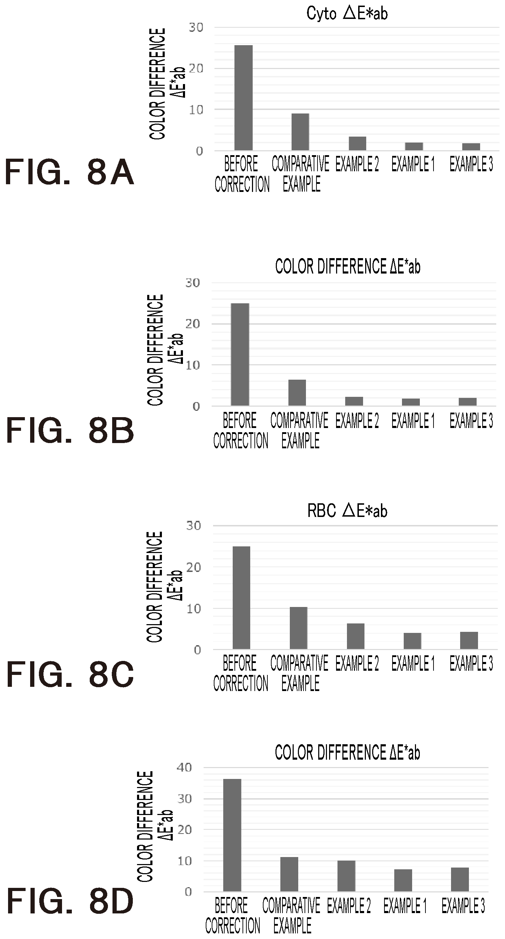

The results are shown in Figs. 8A to 8D. Figs. 8A to 8D are graphs respectively illustrating changes in the color difference ΔE*ab before and after color correction of the images of cytoplasm, extracellular matrix (ECM), red blood cells (RBCs), and cell nuclei.

Referring to Figs. 8A to 8D, the color difference ΔE*ab was reduced when the color correction charts (Examples 1 to 3) were used, as compared with the case where the color correction chart (Comparative Example) was used. In other words, the use of the color correction charts (Examples 1 to 3) made the color-corrected image more similar to the actual stained pathological cell preparation. Specifically, the order of the correction accuracy achieved with the color correction chart was Example 1 ≥ Example 3 > Example 2 > Comparative Example. The color difference ΔE*ab from the original image was improved by performing correction using not only the color patches of the standard colors defined in the BT.709 standard and grayscale color patches but also the color patches of colors located within the first color gamut L1 and the second color gamut L2 on the xy chromaticity diagram. Furthermore, the color difference ΔE*ab from the original image was further improved by performing correction using colors located within the third color gamut L3 on the xy chromaticity diagram.

[Investigation of Correction Accuracy When Using Different WSI Scanners]

The correction accuracy was investigated for the two color correction charts (Example 1 and Comparative Example) when different WSI scanners were used. In this case, a specimen (see Fig. 7) of an actual HE-stained pathological cell preparation was prepared as described above. The specimen of the stained pathological cell preparation was imaged using three types of WSI scanners (NanoZoomer HT1 available from Hamamatsu Photonics K.K.,

NanoZoomer XR1 available from Hamamatsu Photonics K.K., and UFS available from Philips). Next, in the captured original images, five points each were extracted from regions of cytoplasm, extracellular matrix (ECM), and cell nuclei, and color correction was performed on the extracted points using each of the color correction charts (Example 1 and Comparative Example).

Next, for (i) the captured original images and (ii) the images that have been color-corrected using the respective color correction charts (Example 1 and Comparative Example), the color difference $\Delta E^*ab$ from the actual stained pathological cell preparation was measured. The method for calculating the color difference $\Delta E^*ab$ was the same as described above.

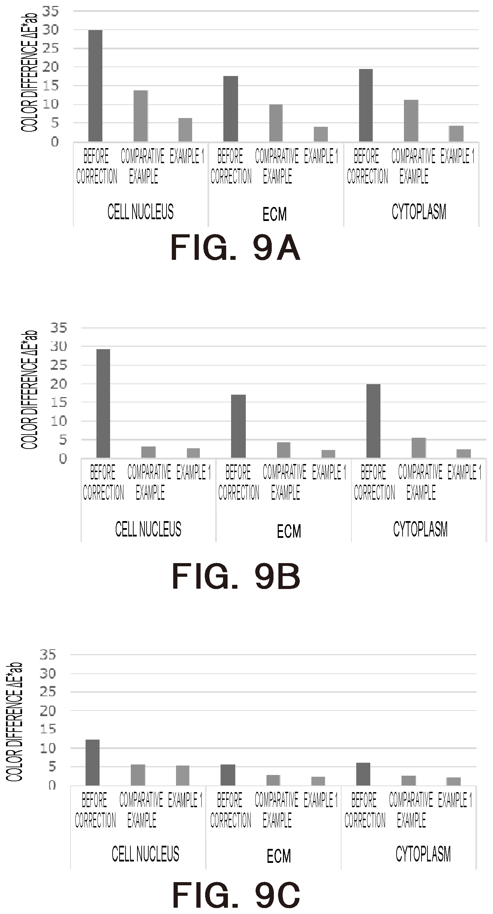

The results are shown in Figs. 9A to 9C.

Fig. 9A illustrates changes in the color difference $\Delta E^*ab$ before and after color correction of the images of cytoplasm, extracellular matrix, and cell nuclei obtained using a WSI scanner (NanoZoomer HT1 available from Hamamatsu Photonics K.K.). Referring to Fig. 9A, when the images captured with the WSI scanner (NanoZoomer HT1 available from Hamamatsu Photonics K.K.) were corrected using the color correction chart (Example 1), the color difference $\Delta E^*ab$ was reduced by 13.8 to 23.6, as compared with the original image of the stained pathological cell preparation.

Fig. 9B illustrates changes in the color difference $\Delta E^*ab$ before and after color correction of the images of cytoplasm, extracellular matrix, and cell nuclei obtained using a WSI scanner (NanoZoomer XR1 available from Hamamatsu Photonics K.K.). Referring to Fig. 9B, when the images captured with the WSI scanner (NanoZoomer XR1 available from Hamamatsu Photonics K.K.) were corrected using the color correction chart (Example 1), the color difference $\Delta E^*ab$ was reduced by 14.8 to 26.5, as compared with the original image of the stained pathological cell preparation.

Fig. 9C illustrates changes in the color difference $\Delta E^*ab$ before and after color correction of the images of cytoplasm, extracellular matrix, and cell nuclei obtained using a WSI scanner (UFS available from Philips). Referring to Fig. 9C, when the images captured with the WSI scanner (UFS available from Philips) were corrected using the color correction chart (Example 1), the color difference $\Delta E^*ab$ was reduced by 3.1 to 6.9, as compared with the original image of the stained pathological cell preparation.

Accordingly, the color difference $\Delta E^*ab$ was reduced by using the color correction chart (Example 1), irrespective of the type of WSI scanner.

[Lightfastness Evaluation]

The following samples were prepared in order to evaluate the lightfastness of the color correction chart.

<Sample Preparation Method>

(Color Patch)

Color patches a to e (Examples) were formed on a substrate composed of a prepress film using water-soluble dyes. The color coordinates and color gamuts of the color patches a to e are shown in the table below.

(Specimen)

Biological tissues stained with biological stains were used as pseudo color patches (Reference Examples). The biological tissues used were from the kidney of a mammal. Staining was performed by a common HE staining method. The color coordinates and color gamuts of the regions evaluated for lightfastness are shown in the table below.

[Table 4]

| | | | Stain /Dye | Color coordinates (x, y) | Corresponding color gamut |
|---|---|---|---|---|---|
| Color Patch (Example) | chart | a | water-soluble dye | (0.323, 0.228) | L3 |
| | chart | b | | (0.306, 0.218) | L3 |
| | chart | c | | (0.280, 0.192) | L2 |
| | chart | d | | (0.364, 0.270) | L4 |
| | chart | e | | (0.320, 0.239) | L3 |

(continued)

| | | | Stain /Dye | Color coordinates (x, y) | Corresponding color gamut |
|---|---|---|---|---|---|
| Pseudo color patch (Reference Example) | specimen (nucleus) | i | biological stain | (0.301, 0.223) | L3 |
| | specimen (cytoplasm) | j | | (0.353, 0.249) | L4 |

[Amount of Color Change (ΔE*ab) After Light Irradiation]

[Lightfastness Evaluation 1]

The color patches a to d and specimens i and j were used as evaluation samples.

The light source was a white LED light source that has a spectral distribution simulating that of standard illuminant D65 and has a color rendering of Ra ≥ 90 and R9 ≥ 80. The color patches a to d were continuously irradiated with light having an irradiance of 53 W/m$^2$, and the specimens i and j were continuously irradiated with light having an irradiance of 62 W/m$^2$ for certain periods of time while keeping the output constant so as to increase the cumulative light dose.

The irradiation time was set to a maximum of 8.2 h for the color patches a to d and a maximum of 7 h for the specimens i and j.

Spectral measurements were performed on the color patches a to d at 0 h, 4 h, and 8.2 h, and on the specimens i and j a total of 14 times at intervals of 15 minutes to 1 hour during the period from 0 h to 7.0 h.

The irradiance was measured using Light Analyzer LA-105 available from Nippon Medical & Chemical Instruments Co., Ltd. (NK System).

The spectra were measured using a spectroradiometer (SR-5000HM, available from Topcon Technohouse Corporation).

The amount of color change ΔE*ab was calculated from the spectra described above, and a straight line connecting the individual ΔE*ab values as a function of cumulative light dose was created for each of the color patches a to d and the specimens i and j.

The ΔE*ab values at the same cumulative light dose were read from the straight lines, and the amounts of color change were compared.

The read amounts of color change ΔE*ab are shown in the table below.

[Table 5]

| Cumulative light dose (W/m$^2$·h) | | | 100 | 200 | 400 |
|---|---|---|---|---|---|
| Color Patch (Example) | chart | a | 0.4 | 0.7 | 1.4 |
| | chart | b | 0.6 | 1.2 | 1.7 |
| | chart | c | 0.6 | 1.2 | 1.6 |
| | chart | d | 0.7 | 1.4 | 2.0 |
| Pseudo color patch (Reference Example) | specimen (nucleus) | i | 2.4 | 3.3 | 5.4 |
| | specimen (cytoplasm) | j | 1.8 | 2.9 | 4.2 |

Referring to the above table, the amounts of color change ΔE*ab of the color patches a to d after irradiation with light at a cumulative light dose of 400 W/m$^2$·h were suppressed to 2.0 or less. The amounts of color change ΔE*ab of the specimens i and j after irradiation with light at a cumulative light dose of 400 W/m$^2$·h reached a maximum of 5.4.

[Lightfastness Evaluation 2]

The amounts of color change in the color patches of Examples after further long-term light irradiation was evaluated.

The color patch e was used as a sample.

The light source was a white LED light source that has a spectral distribution simulating that of standard illuminant D65 and has a color rendering of Ra ≥ 90 and R9 ≥ 80. The color patch e was continuously irradiated with light having an irradiance of 53 W/m$^2$ for a certain period of time while keeping the output constant so as to increase the cumulative light dose.

Spectral measurements were performed at irradiation times of 0 h, 15 h, 30 h, 45 h, and 60 h, and the amount of color change ΔE*ab was calculated.

The ΔE*ab value at 2000 W/m²·h was read from a straight line connecting the individual ΔE*ab values as a function of cumulative light dose. As a result, the ΔE*ab value was 2.1.

[Amount of Change and Rate of Change in Spectrum After Light Irradiation]

For the samples (the color patches a to d, and the specimens i and j) used in [Lightfastness Evaluation 1], the amount of change and the rate of change in the spectra before and after the lightfastness evaluation were calculated.

With the spectrum before irradiation used as a reference, the amount of change (%) and the rate of change (%T) in transmittance at wavelengths of 500 nm and 520 nm were calculated for the spectrum after irradiation with light at a cumulative light dose of 435 W/m²·h.

The results are shown in the table below.

[Table 6]

| | | | 500nm | | 520nm | |
|---|---|---|---|---|---|---|
| | | | Amount of Change (%T) | Rate of Change (%) | Amount of Change (%T) | Rate of Change (%) |
| Color Patch (Example) | chart | a | 0 | 1 | 0 | 0 |
| | chart | b | 0 | 1 | 0 | 1 |
| | chart | c | 1 | 2 | 0 | 0 |
| | chart | d | 1 | 1 | 0 | 1 |
| Pseudo color patch (Reference Example) | specimen (nucleus) | i | 5 | 20 | 4 | 25 |
| | specimen (cytoplasm) | j | 5 | 22 | 4 | 29 |

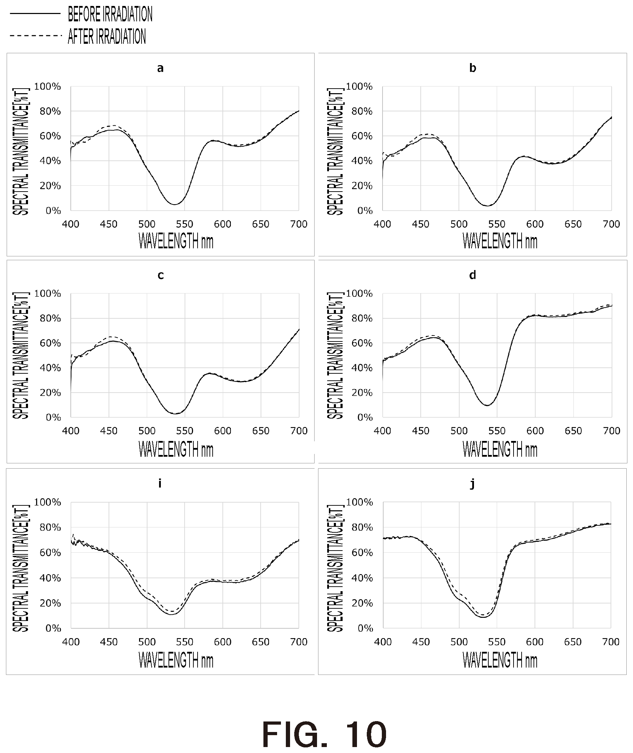

For each of the color patches a to d and the specimens i and j, the spectra before and after the lightfastness evaluation are shown in Fig. 10.

Multiple components disclosed in the embodiments and modifications described above can be combined with each other as needed. Alternatively, some components may be omitted from all of the components shown in the embodiments and modifications described above.

**Claims**

1. A color correction chart comprising:

   a substrate; and
   a color patch formed on the substrate,
   wherein the color patch includes a first color, and
   the first color has a coordinate point within a region bounded by three points (0.200, 0.070), (0.450, 0.280), and (0.310, 0.340) on an xy chromaticity diagram.

2. The color correction chart according to claim 1, wherein the first color has a coordinate point within a region bounded by five points (0.310, 0.340), (0.350, 0.300), (0.350, 0.200), (0.260, 0.150), and (0.280, 0.250) on the xy chromaticity diagram.

3. The color correction chart according to claim 1, wherein the first color has a coordinate point within a region bounded by four points (0.311, 0.334), (0.347, 0.210), (0.270, 0.162), and (0.292, 0.216) on the xy chromaticity diagram.

4. The color correction chart according to claim 1, wherein the first color has a coordinate point within a region bounded by three points (0.350, 0.300), (0.350, 0.200), and (0.380, 0.280) on the xy chromaticity diagram.

5. The color correction chart according to claim 1, further comprising a plurality of color patches formed on the substrate,

 wherein the color patches each have a color different from the first color,
 the color patches each include a different color, and
 the color patches have color coordinate points within the region bounded by three points (0.200, 0.070), (0.450, 0.280), and (0.310, 0.340) on the xy chromaticity diagram.

6. The color correction chart according to claim 5, wherein the color patches have color coordinate points within a region bounded by five points (0.310, 0.340), (0.350, 0.300), (0.350, 0.200), (0.260, 0.150), and (0.280, 0.250) on the xy chromaticity diagram.

7. The color correction chart according to claim 5, wherein the color patches have color coordinate points within a region bounded by four points (0.311, 0.334), (0.347, 0.210), (0.270, 0.162), and (0.292, 0.216) on the xy chromaticity diagram.

8. The color correction chart according to claim 2, further comprising an additional color patch formed on the substrate, wherein the additional color patch has a color coordinate point within a region bounded by three points (0.350, 0.300), (0.350, 0.200), and (0.380, 0.280) on the xy chromaticity diagram.

9. The color correction chart according to claim 1, wherein the first color has a coordinate point outside a white region defined by a blackbody locus of 5000 K or higher and 9300 K or lower and a color deviation of $d_{UV} = \pm 0.005$ from the blackbody locus.

10. The color correction chart according to claim 1, wherein a spectrum of the first color has a peak wavelength of 520 nm or more and 560 nm or less, a point at which the spectrum rises toward a long-wavelength side from the peak wavelength is in a range of 520 nm or more and 560 nm or less, and an increase in spectral transmittance in a range of 560 nm or more and 620 nm or less is 40 %T or less.

11. The color correction chart according to claim 10, wherein the spectral transmittance at the peak wavelength is 0.5 %T or more and 45 %T or less.

12. The color correction chart according to claim 1, wherein an amount of color change $\Delta E^*ab$ of the first color of the color patch is 2.5 or less after irradiation with light from a white LED light source having a color rendering of Ra $\geq$ 90 and R9 $\geq$ 80 and having a spectral distribution simulating that of standard illuminant D65 is performed at an irradiance of 50 W/m$^2$ to 65 W/m$^2$ on an irradiated object and a cumulative light dose of 400 W/m$^2$·h.

13. The color correction chart according to claim 1, wherein at least one of an amount of change in transmittance (%T) at 500 nm and an amount of change in transmittance (%T) at 520 nm in a spectrum of the first color of the color patch is 2.0 %T or less after irradiation with light from a white LED light source having a color rendering of Ra $\geq$ 90 and R9 $\geq$ 80 and having a spectral distribution simulating that of standard illuminant D65 is performed at an irradiance of 50 W/m$^2$ to 65 W/m$^2$ on an irradiated object and a cumulative light dose of 435 W/m$^2$·h.

14. The color correction chart according to claim 1, wherein at least one of a rate of change in transmittance (%T) at 500 nm and a rate of change in transmittance (%T) at 520 nm in a spectrum of the first color of the color patch is 10% or less after irradiation with light from a white LED light source having a color rendering of Ra $\geq$ 90 and R9 $\geq$ 80 and having a spectral distribution simulating that of standard illuminant D65 is performed at an irradiance of 50 W/m$^2$ to 65 W/m$^2$ on an irradiated object and a cumulative light dose of 435 W/m$^2$·h.

15. The color correction chart according to claim 1, wherein the substrate includes a dye-fixing layer.

FIG. 1

FIG. 2A

FIG. 2B

FIG. 2C

FIG. 3A

FIG. 3B

FIG. 3C

FIG. 4

FIG. 5

FIG. 6A

FIG. 6B

FIG. 6C

CYTOPLASM

EXTRACELLULAR MATRIX (ECM)

CELL NUCLEUS

# FIG. 7

FIG. 8A

FIG. 8B

FIG. 8C

FIG. 8D

FIG. 9A

FIG. 9B

FIG. 9C

FIG. 10

**EP 4 779 386 A1**

## INTERNATIONAL SEARCH REPORT

| International application No. |
|---|
| **PCT/JP2024/031975** |

**A. CLASSIFICATION OF SUBJECT MATTER**

*G02B 21/34*(2006.01)i; *G01J 3/52*(2006.01)i; *G01N 21/01*(2006.01)i; *G01N 21/27*(2006.01)i; *G02B 21/00*(2006.01)i
FI: G02B21/34; G01J3/52; G01N21/01 A; G01N21/27 E; G01N21/27 F; G02B21/00

According to International Patent Classification (IPC) or to both national classification and IPC

**B. FIELDS SEARCHED**

Minimum documentation searched (classification system followed by classification symbols)

G02B21/34; G01J3/52; G01N21/01; G01N21/27; G02B21/00

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| X | JP 2013-088226 A (NIPPON HOSO KYOKAI) 13 May 2013 (2013-05-13) paragraphs [0022], [0032]-[0035], fig. 5, etc. | 1-9, 12-15 |
| A | | 10-11 |
| A | WO 2021/075406 A1 (DAI NIPPON PRINTING CO., LTD.) 22 April 2021 (2021-04-22) entire text, all drawings | 1-15 |
| A | JP 2015-102708 A (DAI NIPPON PRINTING CO., LTD.) 04 June 2015 (2015-06-04) entire text, all drawings | 1-15 |
| A | JP 2022-164615 A (FFEI LIMITED) 27 October 2022 (2022-10-27) entire text, all drawings | 1-15 |
| A | WO 2013/186530 A1 (FFEI LIMITED) 19 December 2013 (2013-12-19) entire text, all drawings | 1-15 |
| A | JP 2017-187756 A (DAI NIPPON PRINTING CO., LTD.) 12 October 2017 (2017-10-12) entire text, all drawings | 1-15 |

☑ Further documents are listed in the continuation of Box C.    ☑ See patent family annex.

| | |
|---|---|
| * Special categories of cited documents:<br>"A" document defining the general state of the art which is not considered to be of particular relevance<br>"D" document cited by the applicant in the international application<br>"E" earlier application or patent but published on or after the international filing date<br>"L" document which may throw doubts on priority claim(s) or which is cited to establish the publication date of another citation or other special reason (as specified)<br>"O" document referring to an oral disclosure, use, exhibition or other means<br>"P" document published prior to the international filing date but later than the priority date claimed | "T" later document published after the international filing date or priority date and not in conflict with the application but cited to understand the principle or theory underlying the invention<br>"X" document of particular relevance; the claimed invention cannot be considered novel or cannot be considered to involve an inventive step when the document is taken alone<br>"Y" document of particular relevance; the claimed invention cannot be considered to involve an inventive step when the document is combined with one or more other such documents, such combination being obvious to a person skilled in the art<br>"&" document member of the same patent family |

| Date of the actual completion of the international search | Date of mailing of the international search report |
|---|---|
| **30 September 2024** | **08 October 2024** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | |
| | Telephone No. |

Form PCT/ISA/210 (second sheet) (July 2022)

40

**INTERNATIONAL SEARCH REPORT**

International application No.

**PCT/JP2024/031975**

| C. DOCUMENTS CONSIDERED TO BE RELEVANT | | |
|---|---|---|
| Category* | Citation of document, with indication, where appropriate, of the relevant passages | Relevant to claim No. |
| A | JP 2013-134170 A (NIPPON HOSO KYOKAI) 08 July 2013 (2013-07-08)<br>entire text, all drawings | 1-15 |
| A | WO 2019/078288 A1 (DAI NIPPON PRINTING CO., LTD.) 25 April 2019 (2019-04-25)<br>entire text, all drawings | 1-15 |

Form PCT/ISA/210 (second sheet) (July 2022)

## INTERNATIONAL SEARCH REPORT
### Information on patent family members

International application No.

**PCT/JP2024/031975**

| Patent document cited in search report | | | Publication date (day/month/year) | Patent family member(s) | | | Publication date (day/month/year) |
|---|---|---|---|---|---|---|---|
| JP | 2013-088226 | A | 13 May 2013 | (Family: none) | | | |
| WO | 2021/075406 | A1 | 22 April 2021 | US | 2022/0390283 | A1 | |
| | | | | entire text, all drawings | | | |
| | | | | EP | 4060298 | A1 | |
| | | | | KR | 10-2022-0078698 | A | |
| | | | | CN | 114761772 | A | |
| JP | 2015-102708 | A | 04 June 2015 | (Family: none) | | | |
| JP | 2022-164615 | A | 27 October 2022 | US | 2022/0334032 | A1 | |
| | | | | entire text, all drawings | | | |
| | | | | GB | 2599751 | A | |
| | | | | DE | 102022107667 | A1 | |
| | | | | CN | 115220208 | A | |
| WO | 2013/186530 | A1 | 19 December 2013 | US | 2015/0192765 | A1 | |
| JP | 2017-187756 | A | 12 October 2017 | US | 2019/0137340 | A1 | |
| | | | | entire text, all drawings | | | |
| | | | | WO | 2017/170910 | A1 | |
| | | | | EP | 3454024 | A1 | |
| | | | | CN | 108885135 | A | |
| | | | | KR | 10-2018-0124904 | A | |
| JP | 2013-134170 | A | 08 July 2013 | (Family: none) | | | |
| WO | 2019/078288 | A1 | 25 April 2019 | US | 2020/0284656 | A1 | |
| | | | | entire text, all drawings | | | |
| | | | | EP | 3699563 | A1 | |
| | | | | CN | 111201425 | A | |
| | | | | KR | 10-2020-0071730 | A | |
| | | | | TW | 201929516 | A | |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 10241310 B

### Non-patent literature cited in the description

- New Edition Color Science Handbook [Third Edition. The Color Science Association of Japan. The University of Tokyo Press